# EUROPEAN PATENT SPECIFICATION

(11) **EP 3 494 103 B1**
(45) Date of publication and mention of the grant of the patent: **06.10.2021**
(21) Application number: 17754650.4
(22) Date of filing: 03.08.2017
(51) Int. Cl.: C07C 29/14, C07C 31/135, C07C 33/14, C07C 45/54, C07C 47/21, C07C 47/32, C07C 49/21, C07C 53/134, C07C 57/02

(54) **1-HYDROXYMETHYL-1,2,2,6-TETRAMETHYL-CYCLOHEXANE AND DERIVATIVES THEREOF AND THEIR USE AS AROMA CHEMICALS**
1-HYDROXYMETHYL-1,2,2,6-TETRAMETHYL-CYCLOHEXAN UND DERIVATE DAVON SOWIE DEREN VERWENDUNG ALS AROMACHEMIKALIEN
1-HYDROXYMÉTHYL-1,2,2,6-TETRAMÉTHYL-CYCLOHEXANE ET SES DÉRIVÉS ET LEUR UTILISATION COMME SUBSTANCES CHIMIQUES AROMATIQUES

(30) Priority: 04.08.2016 IN 201621026655; 15.09.2016 EP 16188975
(43) Date of publication of application: 12.06.2019
(73) Proprietor: BASF SE, 67056 Ludwigshafen am Rhein (DE)
(72) Inventor: HICKMANN, Volker, 67063 Ludwigshafen (DE); RUEDENAUER, Stefan, 69469 Weinheim (DE); PELZER, Ralf, 37699 Fuerstenberg (DE); SWAMINATHAN, Vijay, Chennai 600093 (IN); HINDALEKAR, Shrirang, Mumbai 400013 (IN); GUPTE, Nitin, Thane 400610 (IN); ARDEKAR, Sadanand, Kalyan E 421306 (IN); KADAM, Mileen, Mumbai 400072 (IN)
(74) Representative: BASF IP Association
(86) International application number: PCT/EP2017/069637
(87) International publication number: WO 2018/024820

(56) References cited:
- EP-A2- 0 324 111
- LABAR, DANIEL ET AL: "Connective (C-C) route to hindered epoxides and olefins from hindered ketones", JOURNAL OF THE CHEMICAL SOCIETY, CHEMICAL COMMUNICATIONS, vol. 10, 1982, pages 564-566, XP002765508, ISSN: 0022-4936, DOI: 10.1039/C39820000564

## Description

### BACKGROUND

Despite a large number of existing aroma chemicals (fragrances and flavorings), there is a constant need for new components in order to be able to satisfy the multitude of properties desired for extremely diverse areas of application. These include, firstly, the organoleptic properties, i.e. the compounds should have advantageous odiferous (olfactory) or gustatory properties. Furthermore, aroma chemicals should, however, also have additional positive secondary properties, such as e.g. an efficient preparation method, the possibility of providing better sensory profiles as a result of synergistic effects with other fragrances, a higher stability under certain application conditions, a higher extendibility, a better staying power, etc.

WO 2012/174064 discloses a synthesis route on millimole scale for (1R,6S)-1,2,2,6-tetramethylcyclohexanecarbaldehyde (see examples 59a to 59d of WO 2012/174064) starting with (S)-3,7-dimethyloct-6-enal and formaldehyde via (S)-3,7-dimethyl-2-methyleneoct-6-enal, (3S)-2,3,7-trimethyloct-6-enal, and (S)-2,3,7-trimethylocta-1,6-dienyl acetate. This document does not describe derivatives of 1,2,2,6-tetramethyl-cyclohexanecarbaldehyde. It is also silent with a use of the aldehyde or derivatives thereof as aroma chemicals.

Vogt et al., Helvetia Chimica Acta, 37, 1954, 1779-1790, relates to cyclisation reactions of α-methyl geranic acid.

EP 0 324 111 relates to the synthesis of cycloaliphatic aldehydes, which can be used as aroma chemicals.

Labar, et al., J. Chem. Soc., Chem. Commun., 10, 564-566, 1982, relates to a connective (C-C) route to hindered epoxides and olefins from hindered ketones.

JP H04-26646 A relates to the preparation of (3S)-3,7-dimethyl-2-methylene-6-octen-1-al as fragrance, wherein (3S)-N,N-diethyl-3,7-dimethyl-1,6-octadienylamine is reacted with formaldehyde or a formaldehyde forming compound.

Vieira et al., Appl. Catalysis A: General, 466, 208-215, relates to synthesis of fragrance compounds from acyclic monoterpenes, in particular to a rhodium catalyzed hydroformulation and tandem hydroformulation/acetalization of linalool and β-citronellene.

The object of the present invention is to provide new aroma chemicals with advantageous properties. These aroma chemicals should specifically have pleasant odiferous properties. Furthermore, we specifically aimed at structures related to the ionones and damascones. The ionones contain a cyclohexene-ring which carries three methyl groups and an alpha,beta-unsaturated ketone as side-chain. In the damascones, the positions of the keto-group and the double bond in the sidechain are exchanged.

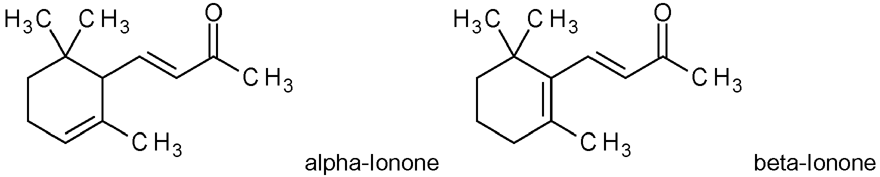

### SUMMARY OF THE INVENTION

The invention provides compounds of formula (A) 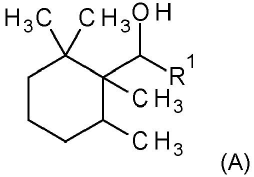
wherein R¹ is selected from C₁-C₈-alkyl, and C₂-C₈-alkenyl,
and esters of the compound of formula (A),
and ketones of the compound of formula (A),
with the proviso that R¹ can also be hydrogen, if compound (A) is an ester of formula (A); and
with the proviso that compound 1-(1,2,2,6-tetramethylcyclohexyl)ethanone is excluded.

In particular, R¹ is selected from C₁-C₄-alkyl, and C₂-C₄-alkenyl, especially from ethyl, n-butyl, 1-propenyl, and 2-propenyl, with the proviso that R¹ can also be hydrogen, if compound (A) is an ester of formula (A).

In a preferred embodiment the invention provides compounds of formula (A) 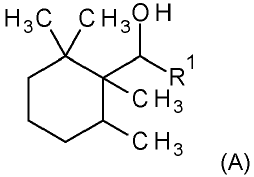
wherein R¹ is selected from C₁-C₄-alkyl, and C₂-C₄-alkenyl, especially from ethyl, n-butyl, 1-propenyl, and 2-propenyl,
and esters of the compound of formula (A),
and ketones of the compound of formula (A),
with the proviso that R¹ can also be hydrogen, if compound (A) is an ester of formula (A); and
with the proviso that compound 1-(1,2,2,6-tetramethylcyclohexyl)ethanone is excluded.

In one aspect of the invention, the esters of the compound of formula (A) are selected from compounds of formula (B), 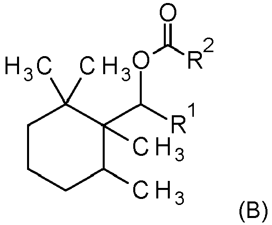 wherein
R¹ is selected from hydrogen, C₁-C₈-alkyl, and C₂-C₈-alkenyl,
R² is selected from C₁-C₅-alkyl.

In particular, R¹ is selected from hydrogen, C₁-C₄-alkyl, C₂-C₃-alkenyl, especially hydrogen, R² is selected from C₁-C₃-alkyl, especially methyl and ethyl.

In one aspect of the invention, the ketones of the compound of formula (A) are selected from compounds of formula (C), 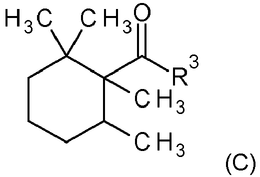 wherein R³ is selected from C₁-C₈-alkyl, and C₂-C₈-alkenyl, with the proviso that R³ is methyl is excluded.

In particular, R³ is selected from C₂-C₄-alkyl, C₂-C₄-alkenyl, especially ethyl, n-butyl, 1-propenyl, 2-propenyl.

The invention further provides a method for preparing compounds of formula (A) 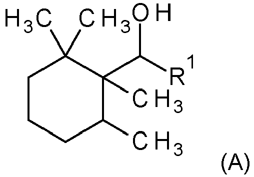 wherein R¹ is selected from hydrogen, C₁-C₈-alkyl, and C₂-C₈-alkenyl,
comprising
i) providing a compound of formula (D), 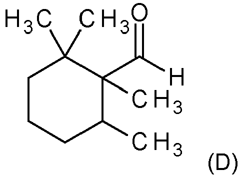 and either
iia) reacting the compound of formula (D) with a reducing agent, which is selected from hydride compounds, or by catalytic hydrogenation, to obtain a compound of formula (A.a), 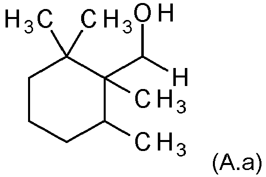 or
iib) reacting the compound of formula (D) with a nucleophilic agent, which is selected from organometallic compounds comprising at least one ligand R³, wherein R³ is selected from C₁-C₈-alkyl and C₂-C₈-alkenyl, to obtain a compound of formula (A.b)

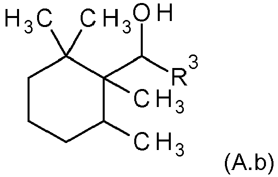

The invention further relates to the use of at least one compound selected from compounds of formula (A) 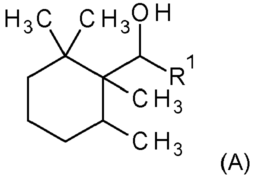
wherein R¹ is selected from hydrogen, C₁-C₈-alkyl, and C₂-C₈-alkenyl,
and the esters of a compound of formula (A) and the ketones of a compound of formula (A) and the compound (D), which are as defined herein, as aroma chemical.

The invention further relates to a flavoring composition comprising
a) at least one compound selected from compounds of formula (A) and the esters of a compound of formula (A) and the ketones of a compound of formula (A), which are as defined herein, and
b) optionally at least one further aroma chemical different from the compounds of component a), and
c) optionally at least one diluent,
with the proviso that the composition comprises at least one of the components b) or c).

The invention further relates to a perfumed or aromatized product,
- comprising an organoleptically effective amount of at least one compound selected from compounds of formula (A) 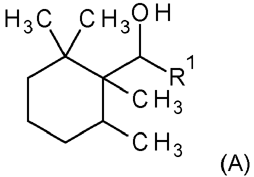
   wherein R¹ is selected from hydrogen, C₁-C₈-alkyl, and C₂-C₈-alkenyl,
   and the esters of a compound of formula (A) and the ketones of a compound of formula (A) and the compound (D), which are as defined herein, and/or
- comprising an organoleptically effective amount of at least one flavoring composition as defined herein.

The invention further relates to a method for imparting and/or intensifying an odor or taste of a product which the product is brought into contact with an organoleptically effective amount of at least one compound selected from compounds of formula (A) 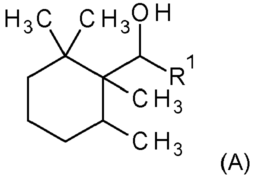
wherein R¹ is selected from hydrogen, C₁-C₈-alkyl, and C₂-C₈-alkenyl,
and the esters of a compound of formula (A) and the ketones of a compound of formula (A) and the compound (D), which are as defined herein.

### DESCRIPTION OF THE INVENTION

The invention has the following advantages:
- The compounds exhibit advantageous aroma properties.
- The compounds are obtainable in large scale and in simple reactions.
- The compounds are easily accessible via an effective synthesis route.
- Introduction of methyl groups adjacent to quaternary carbon atom.
- The synthesis route allows the synthesis without a purification step during the synthesis, which allows easy upscaling / telescoping of the route.

In the context of the present invention, C₁-C₈-alkyl describes an alkyl moiety with 1 to 8 C-atoms, such as methyl, ethyl, n-propyl, isopropyl, n-butyl, isobutyl, sec-butyl, tert-butyl, n-pentyl, n-hexyl, n-heptyl, n-octyl and the structural isomers thereof.

Preferred C₁-C₈-alkyls are C₁-C₄-alkyl, such as methyl, ethyl, n-propyl, isopropyl, n-butyl, isobutyl, sec-butyl, tert-butyl.

In the context of the present invention, C₂-C₈-alkenyl describes an alkenyl moiety with 2 to 8 C-atoms and with one or more, for example 1, 2, 3, or more than 3, C-C unsaturated bonds, such as vinyl, 1-propenyl, 2-propenyl, 1-methyl-vinyl, 1-butenyl, 2-butenyl, 3-butenyl, 1,3-butadienyl, n-butadienyl, n-pentenyl, n-pentadienyl, n-hexenyl, n-hexadienyl, n-heptenyl, n-heptadienyl, n-octenyl, n-octadienyl, and the structural isomers thereof.

Preferred C₂-C₈-alkenyls are C₂-C₄-alkenyls with one or two C-C unsaturated bonds, such as vinyl, 1-propenyl, 2-propenyl, 1-methyl-vinyl, 1-butenyl, 2-butenyl, 3-butenyl, 1,3-butadienyl, and the structural isomers thereof.

In the context of the present invention, R¹ is selected from hydrogen, C₁-C₈-alkyl and C₂-C₈-alkenyl. Preferably, R¹ is selected from hydrogen, C₁-C₄-alkyl and C₂-C₄-alkenyl. In particular, R¹ is selected from hydrogen, ethyl, n-butyl, 1-propenyl and 2-propenyl.

In the context of the present invention, R² is selected from C₁-C₅-alkyl. Preferably, R² is selected from C₁-C₃-alkyl, especially methyl and ethyl.

In the context of the present invention, R³ is selected from hydrogen, C₁-C₈-alkyl and C₂-C₈-alkenyl, with the proviso that R³ is methyl is excluded. Preferably, R³ is selected from hydrogen, C₁-C₄-alkyl and C₂-C₄-alkenyl, with the proviso that R³ is methyl is excluded, in particular, R³ is selected from C₂-C₄-alkyl and C₂-C₄-alkenyl especially ethyl, n-butyl, 1-propenyl, 2-propenyl.

In the context of the present invention, saturated monocarboxylic acid describes a monocarboxylic acid which usually has 1 to 8 C-atoms, such as formic acid, acetic acid, propanoic acid, butanoic acid, hexanoic acid and the isomers thereof.

In the context of the present invention, unsaturated monocarboxylic acid describes a monocarboxylic acid which usually has 3 to 8 C-atoms, such as acrylic acid, butenoic acid, hexenoic acid, heptenoic acid, octenoic acid and the isomers thereof.

In the context of the present invention, the halides of saturated and unsaturated monocarboxylic acids are selected from acid fluoride, acid chloride, and acid bromide of the monocarboxylic acids.

In the context of the present invention, the anhydrides of saturated and unsaturated monocarboxylic acid are selected from anhydrides based on saturated and unsaturated monocarboxylic acids. These can be similar or different. Preferred anhydrides of saturated monocarboxylic acids have the formula (C₁-C₇-alkyl)-C(O)-O-C(O)-(C₁-C₇-alkyl), wherein, preferably, both alkyl moieties are similar.

In the context of the present invention, the C₁-C₄-alkyl esters of saturated and unsaturated monocarboxylic acids are selected from the methyl, ethyl, n-propyl, iso-propyl, n-butyl, sec-butyl, iso-butyl, and tert-butyl esters of saturated and unsaturated monocarboxylic acids.

Unless stated otherwise, the compounds of formulae (A), (B), (C), and (D) include all configurational isomers, in particular all diastereomers and/or enantiomers in pure form and mixtures thereof, optically active mixtures of the enantiomers of these compounds, and also optically inactive racemates.

In the context of the present invention, an organoleptically effective amount is to be understood as an amount which suffices, upon application as intended, to bring about a scent impression for the user or consumer.

### Compounds of formula (A)

The invention relates to compounds of formula (A) as defined herein, wherein R¹ is selected from C₁-C₈-alkyl, and C₂-C₈-alkenyl.

Preferably, R¹ is selected from C₁-C₄-alkyl, and C₂-C₄-alkenyl. More preferably, R¹ is selected from C₁-C₃-alkyl, and C₂-C₃-alkenyl.

R¹ is especially selected from ethyl, n-butyl, 1-propenyl and 2-propenyl.

Particularly preferred compounds of formula (A) are selected from the compounds of formulae (A.2) (A.3) (A.4) and (A.5), in particular (A.2) (A.3) (A.4) and (A.5)

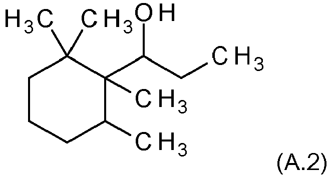

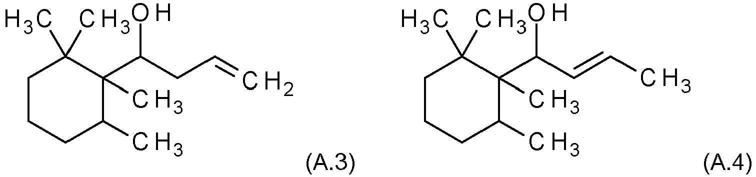

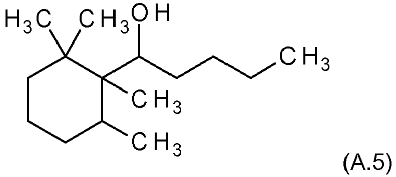

Esters of the compound of formula (A), in particular compounds of formula (B)

The invention also relates to esters of the compounds of formula (A) 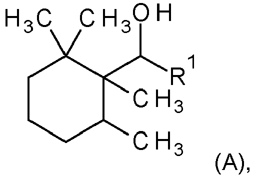 wherein R¹ and R² are defined above. Preferred esters are the ester compounds of formula (B) as defined herein, 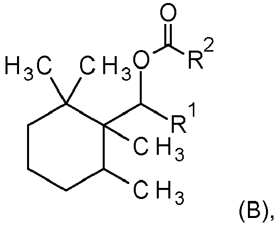 wherein
R¹ is selected from hydrogen, C₁-C₈-alkyl, and C₂-C₈-alkenyl,
R² is selected from C₁-C₅-alkyl.

In particular, R¹ is selected from hydrogen, C₁-C₄-alkyl, C₂-C₃-alkenyl, especially hydrogen, R² is selected from C₁-C₃-alkyl, especially methyl and ethyl.

The esters of the compound of formula (A) are usually obtained by esterification of the hydroxyl group with a compound selected from ketenes, saturated and unsaturated monocarboxylic acids, halides of saturated and unsaturated monocarboxylic acids, anhydrides of saturated and unsaturated monocarboxylic acids, and C₁-C₄-alkyl esters of saturated and unsaturated monocarboxylic acids.

Preferably, the esterification of the hydroxyl group is carried our using a ketene of formula (K) 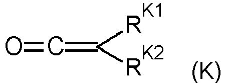 wherein R^{K1} and R^{K2} are independently from each other selected from hydrogen, C₁-C₈-alkyl, and C₂-C₈-alkenyl. Preferably, R^{K1} and R^{K2} are independently from each other selected from hydrogen, C₁-C₈-alkyl, and C₂-C₈-alkenyl, which are more preferably similar.

In a particular preferred embodiment, R^{K1} and R^{K2} are both hydrogen. In this case, the ketene (K) is ethenone.

Ethenone is preferably generated by high temperature pyrolysis of acetone or acetic acid at temperatures generally higher than 650°C. The temperature is preferably in the range from 650 to 1000°C, particularly preferably from 700 to 900°C.

In a specific embodiment, ethenone is prepared under reduced pressure. The pressure is preferably in the range from about 100 to 900 mbar, particularly preferably from 300 to 500 mbar, especially from 350 to 450 mbar. In an alternative embodiment, ethenone is prepared at ambient pressure. In this case, the pressure is preferably in the range from about 950 to 1050 mbar.

Since ketene compounds and particularly ethenone are exceptionally reactive compounds which have a strong tendency to dimerize forming diketenes, a ketene compound is used in the method according to the invention which has preferably been prepared only briefly beforehand. The method according to the invention is rendered particularly advantageous when using ethenone which has been prepared directly prior to the reaction in the method according to the invention, for example, by thermal cleavage of acetone, acetic acid or acetic anhydride.

In a first variant of the method according to the invention, the ketene and in particular ethenone is introduced into the reaction mixture below the liquid surface such that it sparges the reaction mixture. The ketene is advantageously fed into the reaction mixture under intensive stirring, so that no ketene substantially converts into the gas phase in relatively large amounts. The pressure of the ketene must be sufficiently high in order to overcome the hydrostatic pressure of the reaction mixture above the ethenone input, optionally supported by a stream of inert gas, e.g. nitrogen.

The ketene can be introduced via any suitable devices. Good distribution and rapid mixing are important here. Suitable devices are, for example, sparging lances which may be fixed in position or preferably nozzles. The nozzles can be provided at or near the bottom of the reactor. For this purpose, the nozzles may be configured as openings from a hollow chamber surrounding the reactor. However, preference is given to using immersed nozzles with suitable feed lines. A plurality of nozzles can, for example, be arranged in the form of a ring. The nozzles may point upward or downward. The nozzles preferably point obliquely downward.

In a second variant of the method according to the invention, the ketene and in particular ethenone is prepared under reduced pressure and reacted under reduced pressure with the compound of formula (A). The pressure during the preparation and reaction of the ketene is preferably in the range from about 100 to 900 mbar, particularly preferably from 300 to 500 mbar, especially from 350 to 450 mbar.

Methods and apparatuses for preparing ethenone are described, for example, in Organic Syntheses, Coll. Vol. 1, p. 330 (1941) and Vol. 4, p. 39 (1925) and in Chemiker Zeitung [The Chemists Journal] 97, No. 2, pages 67 to 73 (1979). If a ketene compound CR^{K1}R^{K2}=C=O (K) is to be used in the method according to the invention, where R^{K1} and R^{K2} are different from hydrogen, the preparation may in principle be carried out by known methods. These include, for example, the elimination of hydrogen halide from carbonyl halides having an adjacent hydrogen. Such methods are described, for example, in Organikum, VEB Deutscher Verlag der Wissenschaften, 16th Edition, Berlin 1986, Chapter 3.1.5, specifically page 234. The preparation of ketene compounds is also possible by way of the Arndt-Eistert synthesis by reacting a carbonyl halide with diazomethane.

An excess of the ketene can lead to undesired side reactions. Therefore, the reaction of the compound of the general formula (A) with the ketene is preferably carried out using at most equimolar amounts of the ketene.

The compound of formula (A) is preferably reacted with the ketene in such a way that an accumulation of the ketene in the reaction mixture is avoided at all times in the reaction.

The reaction of the compound of the formula (A) with the ketene preferably takes place in such a way that ketene is introduced into the reaction mixture until the conversion of compound of formula (A) is at least 90%, preferably at least 95%.

The compound of the formula (A) is preferably subjected to a reaction with ketene at a temperature in the range of 0 to 150°C, preferably 40 to 120°C and more preferably 80 to 100°C.

In a first preferred embodiment, the compound of the formula (A) is subjected to a reaction with the ketene in the absence of an added catalyst.

In a second preferred embodiment, the compound of the formula (A) is subjected to a reaction with the ketene in the presence of a catalyst. Preference is given to using at least one zinc salt as catalyst which may also be present as a hydrate or polyhydrate.

Particular preference is given to using a zinc salt of a carboxylic acid as catalyst, especially a monocarboxylic acid having 1 to 18 carbon atoms or dicarboxylic acid having 2 to 18 carbon atoms. These include, e.g. zinc formate, zinc acetate, zinc propionate, zinc butyrate, zinc stearate, zinc succinate or zinc oxalate. Particular preference is given to zinc acetate.

It is very advantageous in the method according to the invention that the catalysts generally only have to be used in very small amounts, which makes the method more cost-effective and facilitates the work-up of the reaction mixture. This applies in particular to using a zinc salt as catalyst.

The catalyst is preferably used in an amount of 0.01 to 2% by weight, particularly preferably 0.02 to 0.5% by weight, based on the total amount of the compound (A).

To perform the reaction according to the invention, it is advantageous to proceed in such a way that said reaction is carried out in a suitable reaction vessel comprising, as essential components, a good stirring and/or mixing device, a metering device for ethenone, a heating device to start the reaction and to maintain the reaction temperature during the postreaction, a cooling device to remove the heat of reaction of the exothermic reaction and a vacuum pump.

For an optimal reaction regime, it is advantageous to meter in the ketene such that it is never present in excess in the reaction mixture and that the reaction mixture is always thoroughly mixed.

For an optimal reaction regime, it is further advantageous to avoid adding ketene too rapidly and to clearly determine the end of the reaction, e.g. by monitoring the reaction heat.

It is also possible to detect ketene, for example, by IR spectroscopy by means of the characteristic carbonyl band.

By means of the method according to the invention, it is possible to prepare the compounds of the formula (A) in a technically simple manner in high purities and nevertheless in excellent yields and space-time yields. Since the reactants are essentially completely converted to products, the method according to the invention is characterized by a maximum atom economy.

Preferably, the esters of the compound of formula (A) are selected from compounds of formula (B), wherein
R¹ is selected from hydrogen, C₁-C₈-alkyl, and C₂-C₈-alkenyl,
R² is selected from C₁-C₅-alkyl.

Preferably, R¹ is selected from hydrogen, C₁-C₄-alkyl, and C₂-C₄-alkenyl and R² is selected from C₁-C₃-alkyl, in particular methyl and ethyl. More preferably, R¹ is hydrogen and R² is selected from C₁-C₃-alkyl, in particular methyl and ethyl.

A particularly preferred compound of formula (B) is the compound of formula (B.1) and (B.2). 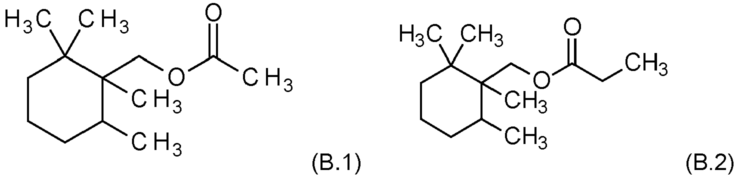 Ketones of the compound of formula (A), in particular compounds of formula (C)

The invention also relates to ketones of the compounds of formula (A) 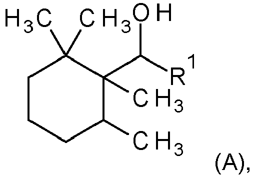 wherein R¹ and R² are defined above. Preferred ketones are the ketones compounds of formula (C) as defined herein, 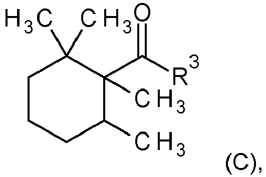 wherein
R³ is selected from C₁-C₈-alkyl, and C₂-C₈-alkenyl, with the proviso that R³ is methyl is excluded.

In particular, R³ is selected from C₂-C₄-alkyl and C₂-C₄-alkenyl, especially from ethyl, n-butyl, 1-propenyl, and 2-propenyl.

The ketones of the compound of formula (A) are usually obtained by oxidation of the hydroxyl group to a keto group.

Usually, the oxidation of the hydroxyl group is carried our using an oxidation reagent which is selected of metal oxides and the salt thereof and oxoacids and the salts thereof.

Examples for metal oxides and the salts thereof are Cr(VI) oxide compounds and its salts, Cr(VI) oxychloro compounds and its salts, and Mn(VII) oxide compounds and its salts, whereby the alkali metal salts, alkaline earth metal salts, and pyridinium salts are preferred.

Examples for oxoacids and the salts thereof are chromic acid and its salts, dichromic acid and its salts, permanganic acid and its salts, manganic acids and its salts, nitric acid and its salts, peroxynitric acid and its salts, phosphoric acid and its salts, sulfiric acid and its salts, disulfuric acid and its salts, peroxomonosulfuric acid and its salts, peroxodisulfuric acid and its salts, perchloric acid and its salts as well as the other analogous halogen compounds thereof, chloric acid and its salts as well as the other analogous halogen compounds thereof, chlorous acid and its salts as well as the other analogous halogen compounds thereof, hypochlorous acid and its salts as well as the other analogous halogen compounds thereof.

Preferably, the oxidation agent is selected of transition metal oxides and the salts thereof, more preferably Cr(VI) oxide compounds and salts thereof and Cr(VI) oxychloro compounds and its salts. In particular, the oxidation agent is pyridinium chlorochromate.

Further preferred oxidation methods are the following:
- oxidation using sodium hypochlorite (NaOCI) in chlorinated solvents using a N-oxide as catalyst, preferably with the use of (2,2,6,6-tetramethylpiperidin-1-yl)-oxidanyl (TEMPO);
- oxidation using aqueous H₂O₂, either in water or in mixtures of water with suitable organic solvents using Mo, W, Mn or V catalyst;
- oxidation using platinum and O₂, in particular PtO₂ and O₂ and/or Pt/C and O₂. A common method is e.g. described by Heyns, Blazejewicz in Tetrahedron, 1960, 9, 67-75.

Preferably, the ketones of the compound of formula (A) are selected from compounds of formula (C), wherein R³ is selected from C₁-C₈-alkyl, and C₂-C₈-alkenyl, with the proviso that R³ is methyl is excluded..

Preferably, R³ is selected from C₁-C₈-alkyl, and C₂-C₈-alkenyl, with the proviso that R³ is methyl is excluded. More preferably, R³ is selected from C₁-C₄-alkyl, and C₂-C₄-alkenyl, with the proviso that R³ is methyl is excluded. Especially, R³ is selected from C₂-C₄-alkyl, and C₂-C₄-alkenyl, especially from ethyl, n-butyl, 1-propenyl, and 2-propenyl.

Particular preferred compounds of formula (C) are selected from compounds of formulae (C.1), (C.2), (C.3) and (C.4).

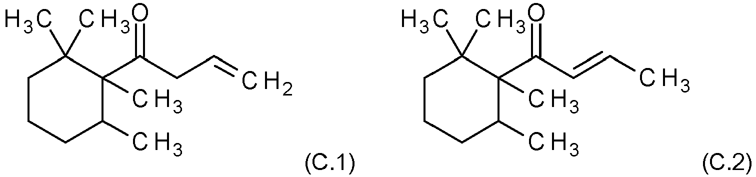

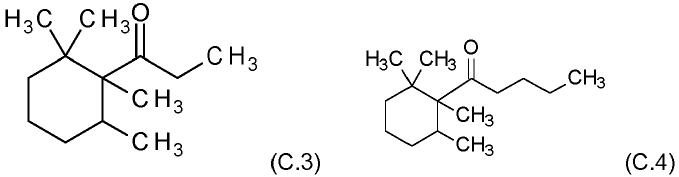

### Method for preparing compounds of formula (A)

The invention also provides a method for preparing compounds of formula (A) comprising
i) providing the compound of formula (D) as defined herein, 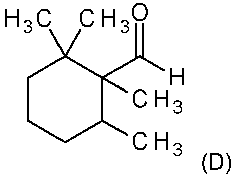 and either
iia) reacting the compound of formula (D) with a reducing agent, which is selected from hydride compounds, or by catalytic hydrogenation, to obtain a compound of formula (A.a),
   or
iib) reacting the compound of formula (D) with a nucleophilic agent, which is selected from organometallic compounds comprising at least one ligand R³, wherein R³ is selected from C₁-C₈-alkyl and C₂-C₈-alkenyl, to obtain a compound of formula (A.b).

Thus, in one embodiment, the invention provides a method for preparing compounds of formula (A.a)
comprising
i) providing a compound of formula (D), and
iia) reacting the compound of formula (D) with a reducing agent, which is selected from hydride compounds, or by catalytic hydrogenation, to obtain a compound of formula (A.a).

In one variant, the aldehyde is reduced with a reducing agent, which is selected from hydride compounds.

Suitable hydride compounds for the reaction in step iia) are for example covalent hydrides, ionic hydrides, metallic hydrides, and transition metal hydride complexes.

Preferably, the hydride compound is selected from boron hydrides, and aluminium hydrides.

In particular, the hydride compound is selected from sodium borohydride (NaBH₄), diisobutylaluminium hydride (DIBAL), sodium bis(2-methoxyethoxy)aluminiumhydride (Red-Al®) and lithium aluminium hydride (LiAlH₄).

With borohydrides, the reaction in step iia) is carried out in an organic solvent such as methanol at temperatures in the range of -20 to +30°C. With aluminium hydrides, the reaction is carried out at temperatures in the range of -30 to +80°C in etheral solvents such as diethyl ether, methyl tert-butyl ether (MTBE), tetrahydrofuran (THF), 2-methyltetrahydrofuran (Me-THF) or toluene. Common reaction conditions are known to persons skilled in the art.

In another variant, the aldehyde is reduced by catalytic hydrogenation.

Suitable catalysts are transition metal catalysts, which are used under an atmosphere of hydrogen. In general, the transition metal is selected from Cu, Ni, Ru, Rh, Pd, and Pt. The catalyst can be either unsupported (e.g. as oxide or, in the case of Raney nickel, as Ni/AI mixture) or supported (0.01 to 10% metal) on carbon, Al₂O₃, ZrO₂, silica, CeO₂, and mixtures thereof. Alternatively, copper-chromite catalysts can be used.

The hydrogen pressure is in general in the range of 2 to 100 bar. Suitable solvents are either alcohols such as methanol or ethanol, esters such as ethyl acetate or etheral solvents such as tetrahydrofuran (THF), 2-methyltetrahydrofuran (Me-THF). Alternatively, the hydrogenation is run without additional solvent.

More specifically, preferred catalytic hydrogenation methods are the following:
a Method analogous to JPH04283527 using copper chromite (Cu/Cr) as catalyst.
b Method analogous to Synlett 2000, 4, 197-200, in particular the reaction of compound 8a to 8b in said article.
c Method using Ru on ZrO₂ (10 wt.-%) as catalyst with up to 100 bar H₂ at up to 150 °C in THF for several hours.
d Method using Pd/C as catalyst in MeOH, optionally containing Me₃N (5 wt.-%), with up to 10 bar H₂ at up to 60°C for several hours.
e Method using Ru/C optionally containing Fe as catalyst in MeOH, optionally containing Me₃N (0.1 to 1 wt.-%), with up to 30 bar H₂ at up to 60°C for several hours.

In a particular aspect, this method further comprises
iiia) reacting the compound of formula (A.a) with a compound selected from ketenes, saturated and unsaturated monocarboxylic acids and halides, anhydrides, and C₁-C₄-alkyl esters thereof, to obtain a compound of formula (B.a), wherein R² is as defined herein.

Thus, the invention also provides a method for preparing compounds of formula (B.a), comprising
i) providing a compound of formula (D), and
iia) reacting the compound of formula (D) with a reducing agent, which is selected from hydride compounds, to obtain a compound of formula (A.a), and
iiia) reacting the compound of formula (A.a) with a compound selected from ketenes, saturated and unsaturated monocarboxylic acids and halides, anhydrides, and C₁-C₄-alkyl esters thereof, to obtain a compound of formula (B.a),
wherein R² is as defined herein.

Usually, the compound selected from ketenes, saturated and unsaturated monocarboxylic acids and halides, anhydrides, and C₁-C₄-alkyl esters thereof as defined hereinabove.

Preferably, the esterification of the hydroxy group is carried out using a ketene. Particular preferred ketenes are as defined hereinabove, specifically preferred is ethenone.

Step iiia) is carried out either without added solvent or in an organic solvent such as toluene. Typically, the temperature is in the range of 70 to 110°C, preferably in the range of 80 to 100°C. Common reaction conditions are known to persons skilled in the art.

In a second embodiment, the invention provides a method for preparing compounds of formula (A.b), comprising
i) providing a compound of formula (D), and
iib) reacting the compound of formula (D) with a nucleophilic agent, which is selected from organometallic compounds comprising at least one ligand R³ to obtain a compound of formula (A.b),
wherein R³ is as defined herein.

Usually, the nucleophilic reagent used in step iib) is selected from metal C₁-C₈-alkyl compounds and metal C₂-C₈-alkenyl compounds, comprising at least one ligand R³ as defined herein. Preferably, the nucleophilic reagent is selected from metal C₁-C₄-alkyl compounds and metal C₂-C₄-alkenyl compounds, comprising at least one ligand R³ as defined herein.

Suitable metals in said compounds are lithium, magnesium, copper, and zinc. Preferred are magnesium and lithium.

In particular, the nucleophilic reagent is selected from MgClR³, MgBrR³, and LiR³, wherein R³ is as defined herein.

Typically, step iib) is carried out in an etheral organic solvent such as tetrahydrofuran and at a temperature in the range of -35 to +15°C. Common reaction conditions are known to persons skilled in the art.

In a particular aspect, this method further comprises
iiib) reacting the compound of formula (A.b) with an oxidation reagent to obtain a compound of formula (C), wherein R³ is as defined herein.

Thus, the invention also provides a method for preparing compounds of formula (C) comprising
i) providing a compound of formula (D), and
iib) reacting the compound of formula (D) with a nucleophilic agent, which is selected from organometallic compounds comprising at least one ligand R³ to obtain a compound of formula (A.b),
iiib) reacting the compound of formula (A.b) with an oxidation reagent to obtain a compound of formula (C),
wherein R³ is as defined herein.

Usually, the oxidation of the hydroxyl group is carried our using an oxidation reagent as defined hereinabove.

Preferably, the oxidizing agent is selected from pyridinium chlorochromate. Typically, step iiib) as carried out in an organic solvent such as dichloromethane and at a temperature in the range of 0 to +30°C. Common reaction conditions are known to persons skilled in the art.

Other suitable oxidation methods are those described above, such as
- oxidation using sodium hypochlorite (NaOCI);
- oxidation using aqueous H₂O₂, either in water or in mixtures of water with suitable organic solvents using Mo, W, Mn or V catalyst; and
- oxidation using platinum and O₂, in particular PtO₂ and O₂ and/or Pt/C and O₂.

### Method for preparing compounds of formula (D)

The invention further provides a method for preparing compounds of formula (A), comprising
ia) providing a compound of formula (D.a), 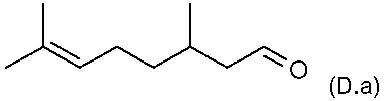
ib) subjecting the compound of formula (D.a) to an aldol condensation reaction to obtain a compound of formula (D.b), 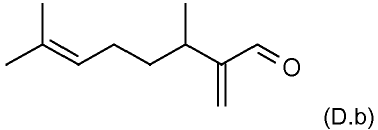
ic) subjecting the compound of formula (D.b) to a hydrogenation reaction to obtain a compound of formula (D.c), 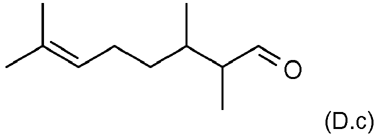
id) subjecting the compound of formula (D.c) to an acetylation reaction to obtain a compound of formula (D.d), 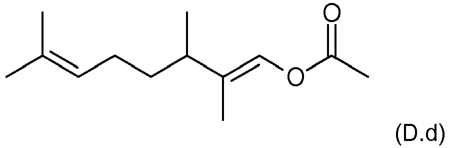
ie) subjecting the compound of formula (D.d) to a cyclisation reaction to obtain the compound of formula (D).

The compound of formula (D.a) is commercially available as "citronellal". The (S)-(-)-Citronellal is available under CAS 5949-05-3, the (R)-(+)-Citronellal is available under CAS 2385-77-5, and a (+-)-Citronellal is available under CAS 106-23-0.

For step ib), general aldol condensation reaction are known to skilled persons.

Usually, the aldol condensation reaction in step ib) is carried out using aqueous formaldehyde. The reaction is usually carried out at temperatures in the range of 10 to 60°C using isopropanol / water as solvent.

For step ic), general hydrogenation reactions are known to skilled persons.

Usually, the hydrogenation reaction in step ic) is carried out using molecular hydrogen in the presence of a catalyst. Preferably, palladium on carbon, Pd/C, is used as catalyst in step ic).

For step id), general acetylation reactions are known to skilled persons.

Typically, the acetylation reaction in step id) is carried out using an acylating agent. Common acylating agents are known to a skilled person. Preferably, acetic anhydride is used in step id).

For step ie), general cyclisation reactions are known to skilled persons.

Typically, the cyclisation reaction in step ie) is performed in the presence of a mineral acid in toluene. Preferably, a mineral acid such as sulfuric acid, nitric acid, phosphoric acid, hydrochloric acid is used in step ie). More preferably, phosphoric acid is used.

### Aroma chemicals

The invention further provides the compounds of the formulae (A) 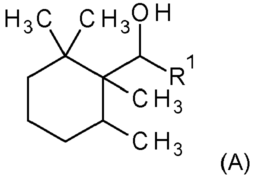 wherein R¹ is selected from hydrogen, C₁-C₈-alkyl, and C₂-C₈-alkenyl; (B), (C) and (D), preferably (A), (B) and (C), as defined herein as aroma chemicals. Preferred compounds to be used as aroma chemicals are those described herein, in particular the compounds selected from compounds of formulae (A.1) (see below definition), (A.2), (A.3), (A.4), (A.5), (B.1), (B.2), (C.1), (C.2), (C.3) and (C.4), particularly (A.1), (A.2), (A.3), (A.4), (A.5), (B.1), (C.1), (C.2) and (C.3). Especially preferred compounds are (A.1), (A.2), (A.3), (B.1), (C.1), (C.2), and (C.3).

An aroma chemical, also known as aroma compound, odorant, aroma, fragrance, or flavor, is a chemical compound that has a smell or odor. A chemical compound typically has a smell or odor when it is sufficiently volatile to be transported to the olfactory system, typically in the upper part of the nose.

The invention provides the use of a compound of formula (A.1) 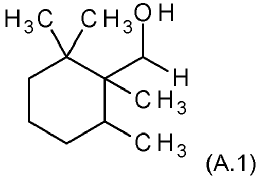 as aroma chemical, in particular for producing a scent or for imparting and/or intensifying an odor or taste of a product with a note and/or aroma of eucalyptol, flowery, earth-like.

The invention provides the use of a compound of formula (A.2) 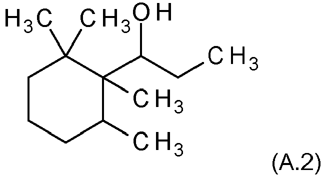 as aroma chemical, in particular for producing a scent or for imparting and/or intensifying an odor or taste of a product with a note and/or aroma of ambra, naphthalene.

The invention provides the use of a compound of formula (A.3) 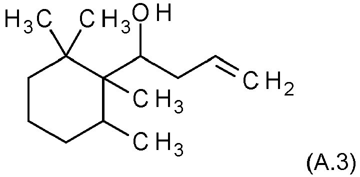 as aroma chemical, in particular for producing a scent or for imparting and/or intensifying an odor or taste of a product with a note and/or aroma of woody, cedar, oak moss.

The invention provides the use of a compound of formula (B.1) 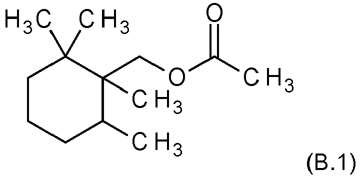 as aroma chemical, in particular for producing a scent or for imparting and/or intensifying an odor or taste of a product with a note and/or aroma of woody, sweet-powdery, fresh, freesia.

The invention provides the use of a compound of formula (C.1) 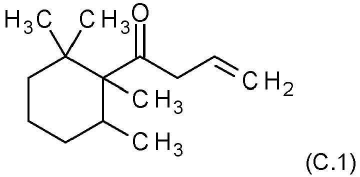 as aroma chemical, in particular for producing a scent or for imparting and/or intensifying an odor or taste of a product with a note and/or aroma of straw, hay, earth-like, slightly woody.

The invention provides the use of a compound of formula (C.2) 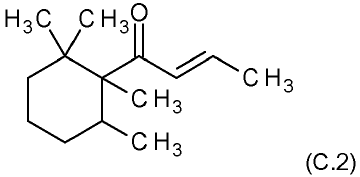 as aroma chemical, in particular for producing a scent or for imparting and/or intensifying an odor or taste of a product with a note and/or aroma of dried fruit, damascone, rosy, lovage.

The invention provides the use of a compound of formula (C.3) 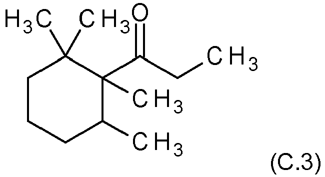 as aroma chemical, in particular for producing a scent or for imparting and/or intensifying an odor or taste of a product with a note and/or aroma of earth-like, cedar wood, powdery, dry.

The disclosure provides the use of a compound of formula (D) 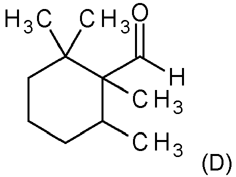 as aroma chemical, in particular for producing a scent or for imparting and/or intensifying an odor or taste of a product with a note and/or aroma of eucalyptol, campher, citrus, spruce needle.

The disclosure describes the use of a compound of formula (D.b) 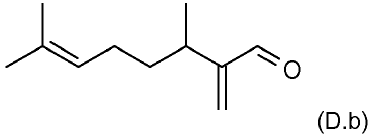 as aroma chemical, in particular for producing a scent or for imparting and/or intensifying an odor or taste of a product with a note and/or aroma of citrus.

The disclosure describes the use of a compound of formula (D.c) 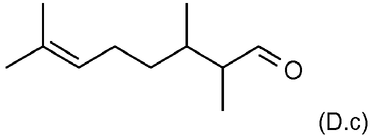 as aroma chemical, in particular for producing a scent or for imparting and/or intensifying an odor or taste of a product with a note and/or aroma of rosy (room filling), citrus, waxy.

Accordingly, the compounds selected from compounds of formulae (A), (B), (C), and (D), preferably selected from compounds of formulae (A), (B), and (C), in particular the preferred compounds as mentioned herein, can be used as aroma chemical or in a flavoring composition as defined herein as agent for providing a product and/or composition with a note of an distinct aroma as described above.

The use as aroma chemical herein comprises in particular the use in products or compositions selected from scent dispensers, fragrances perfumes, perfumes, detergents and cleaners, cosmetic compositions, bodycare compositions, hygiene articles, products for oral and dental hygiene.

The use as aroma chemical herein further comprises the use in products and in perfumed articles as mentioned below.

### Flavoring compositions

The invention further provides a flavoring composition comprising
a) at least one compound selected from compounds of formulae (A), (B), (C), and (D), preferably selected from compounds of formulae (A), (B), and (C),
b) optionally at least one further aroma chemical different from the compounds of component a), and
c) optionally at least one diluent,
with the proviso that the composition comprises at least one of the components b) or c).

In a preferred embodiment, the flavoring composition according to the invention comprises the component a) as the sole aroma chemical.

In a further preferred embodiment, the flavoring composition according to the invention comprises at least one further aroma chemical b) different from the compound of formulae (A), (B), (C), and (D).

Further aroma chemicals, flavors and specifically odorants can be found e.g. in S. Arctander, Perfume and Flavor Chemicals, Vol. I and II, Montclair, N. J., 1969, self published or K. Bauer, D. Garbe and H. Surburg, Common Fragrance and Flavor Materials, 4th. Ed., Wiley-VCH, Weinheim 2001.

Preferably, the quantitative weight ratio of component a) to component b) is in a range from 100:1 to 1:100, particularly preferably from 50:1 to 1:50.

The aroma chemical or flavoring composition can optionally comprise at least one diluent c). Suitable diluents can be used individually or as a mixture of 2 or more than 2 diluents. Suitable diluents are those as are customarily used as solvents for flavoring compositions, fragrances, or flavors.

Preferably, the flavoring compositions comprise as diluents c), at least one compound which is liquid at 20°C and 1013 mbar.

Preferably, the compounds of component a) have a solubility in component c) at 20°C of at least 0.1 mg/ml, particularly preferably of at least 0.5 mg/ml. Preferably, if present, the compounds of component b) have a solubility in component c) at 20°C of at least 0.1 mg/ml, particularly preferably of at least 0.5 mg/ml.

Component c) is preferably selected from aliphatic and cycloaliphatic monoalcohols, polyols, open-chain aliphatic ethers, cyclic ethers, polyol mono- and polyethers, esters and mixtures thereof.

Suitable aliphatic and cycloaliphatic monoalcohols are e.g. ethanol, n-propanol, isopropanol, n-butanol, sec-butanol, tert-butanol and cyclohexanol. Suitable polyols are ethylene glycol, propylene glycol, 1,2-butylene glycol, diethylene glycol, dipropylene glycol or glycerol. Suitable open-chain aliphatic ethers and cyclic ethers are e.g. diethyl ether, dipropyl ether, diisopropyl ether, methyl tert-butyl ether, tetrahydrofuran, tetrahydropyran, 1,4-dioxane or morpholine. Suitable polyol mono- and polyethers are e.g. ethylene glycol monomethyl ether, ethylene glycol dimethyl ether, ethylene glycol monoethyl ether, ethylene glycol diethyl ether, propylene glycol monoethyl ether, propylene glycol diethyl ether or diethylene glycol monoethyl ether. Suitable esters are ethyl acetate, methyl acetate, propyl acetate, isopropyl acetate, n-butyl acetate, sec-butyl acetate, tert-butyl acetate, isobutyl acetate, isoamyl acetate, ethyl butyrates, ethyl lactate, diethyl carbonate, ethylene carbonates, propylene carbonate, triethyl citrate, isopropyl myristate, diethyl phthalate, dialkyl esters of 1,2-cyclohexanedicarboxylic acid, specifically 1,2-cyclohexanedicarboxylic acid diisononyl ester (Hexamoll ® DINCH, BASF SE), and the like.

### Perfumed or aromatized product

The invention further provides a perfumed or aromatized product,
- comprising an organoleptically effective amount of at least one compound selected from compounds of formulae (A) 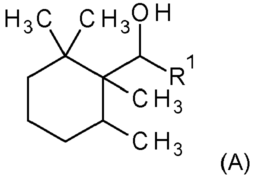 wherein R¹ is selected from hydrogen, C₁-C₈-alkyl, and C₂-C₈-alkenyl; (B), (C), and (D) as defined herein, preferably selected from compounds of formulae (A), (B), and (C), and/or
- comprising an organoleptically effective amount of at least one flavoring composition as defined herein.

A compound of formula (A), (B), (C), and (D) according to the invention and used according to the invention can be incorporated into a series of products and/or be applied to such products such as scent dispensers, fragrances perfumes, perfumes, detergents and cleaners, cosmetic compositions, bodycare compositions, hygiene articles, products for oral and dental hygiene.

Aroma chemicals according to the invention can be used in the production of perfumed articles. The olfactory properties, like the material properties (such as solubility in customary solvents and compatibility with further customary constituents of such products) of the aroma chemicals according to the invention underline their particular suitability for the stated use purposes. The positive properties contribute to the fact that the aroma chemicals used according to the invention and the flavoring compositions according to the invention are particularly preferably used in perfume products, body care products, hygiene articles, textile detergents, and in cleaners for solid surfaces.

The perfumed article is e.g. selected from perfume products, body care products, hygiene articles, textile detergents and cleaners for solid surfaces. Preferred perfumed articles according to the invention are also selected from among:
- perfume products selected from perfume extracts, Eau de Parfums, Eau de Toilettes, Eau de Colognes, Eau de Solide, Extrait Parfum, air fresheners in liquid form, gel-like form or a form applied to a solid carrier, aerosol sprays, scented cleaners and oils;
- body care products selected from aftershaves, pre-shave products, splash colognes, solid and liquid soaps, shower gels, shampoos, shaving soaps, saving foams, bath oils, cosmetic emulsions of the oil-in-water type, of the water-in-oil type and of the water-in-oil-in-water type, such as e.g. skin creams and lotions, face creams and lotions, sunscreen creams and lotions, aftersun creams and lotions, hand creams and lotions, foot creams and lotions, hair removal creams and lotions, aftershave creams and lotions, tanning creams and lotions, hair care products such as e.g. hairsprays, hair gels, setting hair lotions, hair conditioners, hair shampoo, permanent and semipermanent hair colorants, hair shaping compositions such as cold waves and hair smoothing compositions, hair tonics, hair creams and hair lotions, deodorants and antiperspirants such as e.g. underarm sprays, roll-ons, deodorant sticks, deodorant creams, products of decorative cosmetics such as e.g. eyeshadows, nail varnishes, make-ups, lipsticks, mascara, toothpaste, dental floss;
- hygiene articles selected from candles, lamp oils, joss sticks, insecticides, repellents, propellants, rust removers, perfumed freshening wipes, armpit pads, baby diapers, sanitary towels, toilet paper, cosmetic wipes, pocket tissues, dishwasher deodorizer;
- cleaners for solid surfaces selected from perfumed acidic, alkaline and neutral cleaners, such as e.g. floor cleaners, window cleaners, dishwashing detergents, bath and sanitary cleaners, scouring milk, solid and liquid toilet cleaners, powder and foam carpet cleaners, waxes and polishes such as furniture polishes, floor waxes, shoe creams, disinfectants, surface disinfectants and sanitary cleaners, brake cleaners, pipe cleaners, limescale removers, grill and oven cleaners, algae and moss removers, mold removers, facade cleaners;
- textile detergents selected from liquid detergents, powder detergents, laundry pretreatments such as bleaches, soaking agents and stain removers, fabric softeners, washing soaps, washing tablets.

It is preferred if the perfumed article is one of the following:
- an acidic, alkaline or neutral cleaner which is selected in particular from all-purpose cleaners, floor cleaners, window cleaners, dishwashing detergents, bath and sanitary cleaners, scouring milk, solid and liquid toilet cleaners, powder and foam carpet cleaners, liquid detergents, powder detergents, laundry pretreatments such as bleaches, soaking agents and stain removers, fabric softeners, washing soaps, washing tablets, disinfectants, surface disinfectants,
- an air freshener in liquid form, gel-like form or a form applied to a solid carrier or as an aerosol spray,
- a wax or a polish, which is selected in particular from furniture polishes, floor waxes and shoe creams, or
- a body care composition, which is selected in particular from shower gels and shampoos, shaving soaps, shaving foams, bath oils, cosmetic emulsions of the oil-in-water type, of the water-in-oil type and of the water-in-oil-in-water type, such as e.g. skin creams and lotions, face creams and lotions, sunscreen creams and lotions, aftersun creams and lotions, hand creams and lotions, foot creams and lotions, hair removal creams and lotions, aftershave creams and lotions, tanning creams and lotions, hair care products such as e.g. hairsprays, hair gels, setting hair lotions, hair conditioners, permanent and semipermanent hair colorants, hair shaping compositions such as cold waves and hair smoothing compositions, hair tonics, hair creams and hair lotions, deodorants and antiperspirants such as e.g. underarm sprays, roll-ons, deodorant sticks, deodorant creams, products of decorative cosmetics.

Ingredients with which aroma chemicals used according to the invention or flavoring compositions according to the invention can preferably be combined are, for example: preservatives, abrasives, antiacne agents, agents to combat skin aging, antibacterial agents, anticellulite agents, antidandruff agents, anti-inflammatory agents, irritation-preventing agents, irritation-alleviating agents, antimicrobial agents, antioxidants, astringents, sweat-inhibiting agents, antiseptics, antistatics, binders, buffers, carrier materials, chelating agents, cell stimulants, cleaning agents, care agents, hair removal agents, surface-active substances, deodorizing agents, antiperspirants, emollients, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant substances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-Calming agents, skin-Cleansing agents, skin care agents, skin-healing agents, skin lightening agents, skin-protective agents, skin-softening agents, cooling agents, skin-Cooling agents, warming agents, skin-warming agents, stabilizers, UV-absorbent agents, UV filters, detergents, fabric softeners, suspending agents, skin-tanning agents, thickeners, vitamins, oils, waxes, fats, phospholipids, saturated fatty acids, mono- or polyunsaturated fatty acids, α-hydroxy acids, polyhydroxy fatty acids, liquefiers, dyes, color-protection agents, pigments, anticorrosives, aromas, flavorings, odorants, polyols, surfactants, electrolytes, organic solvents or silicone derivatives.

According to a further aspect, the aroma chemicals are used in the production of the perfumed articles in liquid form, undiluted or diluted with a solvent or in the form of a flavoring composition. Suitable solvents for this purpose are those mentioned above as component c). Reference is made hereto in their entirety.

The aroma chemicals and/or flavoring compositions present in the perfumed articles according to the invention can in this connection, in one embodiment, be absorbed onto a carrier, which ensures both fine distribution of the odorant or odorant composition within the product and controlled release upon use. Carriers of this type may be porous inorganic materials such as light sulfate, silica gels, zeolites, gypsums, clays, clay granules, aerated concrete, etc. or organic materials such as woods and cellulose-based materials.

The aroma chemicals used according to the invention and the flavoring compositions according to the invention can also be in microencapsulated form, spray-dried form, in the form of inclusion complexes or in the form of extrusion products and be added in this form to the product or article to be perfumed. The properties can be further optimized by so-called "coating" with suitable materials with regard to a more targeted release of the scent, for which purpose preferably waxy synthetic substances such as e.g. polyvinyl alcohol are used.

The microencapsulation can take place for example by the so-called coacervation method with the help of capsule materials, e.g. made of polyurethane-like substances or soft gelatin. The spray-dried perfume oils can be produced for example by spray-drying an emulsion or dispersion comprising the perfume oil, wherein carrier substances that can be used are modified starches, proteins, dextrin and vegetable gums. Inclusion complexes can be prepared e.g. by introducing dispersions of odorant compositions and cyclodextrins or urea derivatives into a suitable solvent, e.g. water. Extrusion products can be produced by melting odorants used according to the invention and odorant compositions according to the invention with a suitable wax-like substance and by extrusion with subsequent solidification, optionally in a suitable solvent, e.g. isopropanol.

### EXAMPLES

### Analysis of odor

The odor of the compounds was determined by smelling / sniffing the sample on a smelling strip. If indicated, the number quantifies the intensity in the range of (1) = very low to (6) = very high.

### Example 1: Compound (D.b)

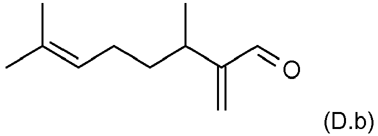

To a solution of citronellal (10.0 kg, 64.9 mol), aqueous formaldehyde (37%, 6.0 kg, 73.8 mol) and isopropanol (iPrOH) (1.04 L), pyrrolidine (550 mL, 6.7 mol) was added over a period of 45 minutes at room temperature. Then, propanoic acid (503 g, 6.7 mol) was added over a period of 45 minutes. After complete addition, the reaction mixture was heated to 45°C for 4 h, cooled back to room temperature, and diluted with water (20.0 L). The phases were separated and the aqueous layer was extracted with ethyl acetate (20.0 L). The combined organic phases were first washed with 5% aqueous NaHCO₃-solution (7.5 L) and then with demineralized water (10 L). The organic layer was separated and the solvent was removed under reduced pressure. The product was obtained as a slightly yellow oil (11 kg, GC purity 98%, yield 99%).
¹H-NMR (500 MHz, CDCl₃): δ = 9.5 (s, 1 H), 6.3 (s, 1 H), 6.0 (s, 1 H), 5.07 - 5.03 (m, 1 H), 2.71 - 2.64 (m, 1 H), 1.96 - 1.84 (m, 2 H), 1.64 (s, 3 H), 1.54 (s, 3 H), 1.54 - 1.47 (m, 1 H), 1.39 - 1.32 (m, 1 H), 1.04 (d, *J* = 7.0 Hz, 3 H) ppm.
¹³C-NMR (125 MHz, CDCl₃): δ = 17.5, 19.4, 25.6, 25.7, 30.8, 35.5, 124.0, 131.4, 132.9, 155.3, 194.4 ppm.

Odor: citrus.

### Example 2: Compound (D.c)

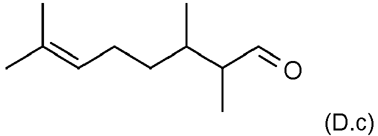

Compound (D.b) (3000 g, 18.04 mol), methanol (832.5 g) and Pd/C (35.1 g, 50% H₂O) were placed in an autoclave under N₂ atmosphere. Trimethylamine (277.3 g, 4.69 mol) was added and the mixture was stirred at room temperature for 30 min before it was heated to 70°C. Then, the autoclave was pressurized with hydrogen (8 bar) and stirring was continued for 29 h, during which 382 L of hydrogen were consumed. After cooling, the autoclave was purged with nitrogen and the reaction mixture was filtered to remove the catalyst. Then, the mixture was diluted with MTBE (2-Methoxy-2-methylpropane, methyl tert-butyl ether) (1.5 L) and extracted with water (1.5 L) three times. The organic phase was dried over Na₂SO₄, filtered again and the solvent was removed under reduced pressure (60°C, 350 mbar). The crude product was obtained in 95% purity and could directly be used in the next step (yellow oil, 2926 g, 92%).
¹H-NMR (500 MHz, CDCl₃, mixture of isomers): δ = 9.67 (d, *J=* 1.9 Hz, 1 H), 9.65 (d, *J* = 1.4 Hz, 1 H), 5.12 - 5.06 (m, 2 H), 2.38 - 2.27 (m, 2 H), 2.09 - 1.90 (m, 6 H), 1.69 - 1.68 (br dd, *J=* 1.1, 4.7 Hz, 6 H), 1.60 (d, *J= 4.7* Hz, 6 H), 1.44 - 1.18 (m, 4 H), 1.04 (d, *J=* 7.0 Hz, 3 H), 1.00 (d, *J=* 2.9 Hz, 3 H), 0.99 (d, *J=* 2.8 Hz, 3 H), 0.84 (d, *J=* 6.9 Hz, 3 H) ppm.
¹³C-NMR (125 MHz, CDCl₃, isomer 1): δ = 7.9, 15.2, 17.4, 25.60, 25.64, 32.0, 34.7, 50.4, 123.9, 131.6, 205.35 ppm.
¹³C-NMR (125 MHz, CDCl₃, isomer 2): δ = 9.7, 17.2, 17.5, 25.56, 25.59, 33.1, 33.2, 51.4, 124.0, 131.6, 205.44 ppm.
Odor: rosy (room filling), citrus, waxy.

### Example 3: Compound (D.d)

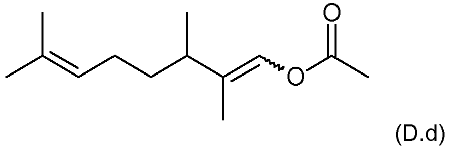

Ac₂O (971.1 g, 9.52 mol), KOAc (93.4 g, 0.95 mol) and Et₃N (1203 g, 11.8 mol) were placed in a double jacketed glass reactor at room temperature. Compound (D.c) (97.6% purity, 820 g, 4.76 mol) was added over a period of 30 min. Then, the reaction mixture was heated to 105 °C and stirring was continued for 6 h. After cooling to 40°C, water (1.5 L) was slowly added with cooling and the mixture was diluted with CH₂Cl₂ (1.5 L). Phases were separated, the aqueous layer was extracted again with CH₂Cl₂ (500 mL) and the combined organic phases were extracted with saturated aqueous NaHCO₃-solution (1 L) and dried over Na₂SO₄. The solvent was removed (50°C, 350 mbar) and the desired product was obtained as a 4:1 E/Z-mixture which could be used directly in the next step (990 g, purity 97%, yield 96%).
¹H-NMR (500 MHz, CDCl₃, mixture of isomers): δ = 6.96 - 6.94 (m, 1 H), 6.86 - 6.84 (m, 1 H), 5.12 - 5.06 (m, 2 H), 2.13 (s, 3 H), 2.11 (s, 3 H), 1.88 (br q, J = 7.4 Hz, 2 H), 1.67 (br d, J = 1.0 Hz, 6 H), 1.60 (d, J = 1.5 Hz, 3 H), 1.58 (br s, 6 H), 1.53 (d, J = 1.6 Hz, 3 H), 1.44 - 1.27 (m, 8 H), 1.01 (d, J = 6.9 Hz, 3 H), 0.98 (d, J = 6.9 Hz, 3 H) ppm.
E-Enolacetate: ¹³C-NMR (125 MHz, CDCl₃): δ = 9.6, 17.7, 19.6, 20.8, 25.7, 26.0, 34.7, 36.9, 125.4, 130.1, 131.4, 124.4, 168.2 ppm.
Z-Enolacetate: ¹³C-NMR (125 MHz, CDCl₃): δ = 12.7, 17.6, 18.6, 20.8, 25.7, 26.1, 31.5, 34.4, 124.6, 125.5, 129.5, 131.3, 168.2 ppm.

### Example 4: Compound (D)

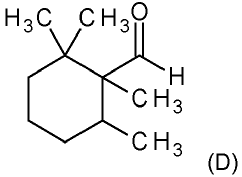

Compound (D.d) (990.0 g,4.6 mol) and toluene (1800 g) were place in a double jacketed glass reactor and heated to 70°C. 85% H₃PO₄ (3400 g) was added over the period of 45 min and stirring was continued for 5 h at 100°C. Then, the biphasic reaction mixture was cooled to 30°C, diluted with water (2 L) and phases were separated. The organic phase was washed with saturated aqueous NaHCO₃-solution (2.5 L), water (1 L) and brine (500 mL). Then, it was dried over Na₂SO₄ and the solvent was removed under reduced pressure. The crude product (753 g, purity 67%) was purified by fractional distillation and obtained as a sticky solid (280 g, purity 93%, yield 34%).
¹H-NMR (500 MHz, CDCI3): δ = 9.61 (s, 1 H), 2.35 (dqd, J= 13.5, 6.8, 3.6 Hz, 1 H), 1.60 - 1.46 (m, 4 H), 1.18 -1.10 (m, 2 H), 1.12 (s, 3 H), 0.94 (s, 3 H), 0.83 (s, 3 H), 0.73 (d, *J* = 6.8 Hz, 3 H).
¹³C-NMR (125 MHz, CDCl₃): δ = 8.2, 17.9, 21.7, 22.8, 27.1, 29.3, 30.6, 36.5, 36.8, 54.2, 209.7 ppm.
Odor: eucalyptol, campher (4), citrus (2), spruce needle (2).

### Example 5: Compound (A.1)

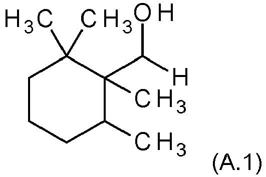

Compound (D) (20 g, 119 mmol) was dissolved in methanol (50 mL) at 0°C. NaBH₄ (4.50 g, 119 mmol) was added portionwise over 30 min. Stirring was continued at 0°C for 1 h after complete addition of NaBH₄. Then, the reaction mixture was diluted with toluene (50 mL) and extracted with brine (30 mL). The organic phase was washed with saturated aqueous NaHCO₃-solution, dried over Na₂SO₄ and the solvent was evaporated under reduced pressure (60°C, 100 mbar). The product was obtained as a low melting solid (17.1 g, purity of crude material 95%, yield 80%).
¹H-NMR (500 MHz, CDCl₃): δ = 3.65 (d, *J=* 11.8 Hz, 1 H), 3.43 (d, *J=* 11.8 Hz, 1 H), 1.93 (dtd, *J* = 13.4, 6.7, 3.7 Hz, 1 H), 1.57 - 1.38 (m, 4 H), 1.28 - 1.16 (m, 2 H), 1.03 (s, 3 H), 0.88 (s, 3 H), 0.86 (d, *J* = 6.8 Hz, 3 H), 0.71 (s, 3 H) ppm.
¹³C-NMR (125 MHz, CDCl₃): δ = 11.9, 17.1, 22.3, 23.8, 26.8, 31.1, 31.6, 36.1, 38.6, 42.1, 67.2 ppm.
Odor: eucalyptol (2), flowery (2), earth-like (2).

### Example 6: Compound (B.1)

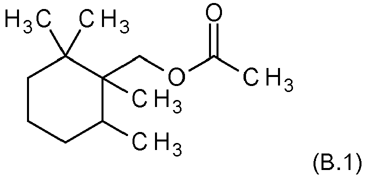

Compound (A.1) (7.9 g, 46.4 mmol) was dissolved in toluene (100 mL) and Zn(OAc)₂ · H₂O (4 mg) was added. Ketene was obtained through the pyrolysis of acetone (0.411 mL/min) at 700°C. The crude ketene-containing gas stream was passed through the reaction mixture at 90°C under vigorous stirring for 3 h. Then, the reactor was purged with nitrogen and cooled to room temperature. The reaction mixture was washed with water and phases were separated. The solvent of the organic phase was removed under reduced pressure and the residue was purified by column chromatography. The product was obtained as a clear liquid (6.5 g, 66%).
¹H-NMR (500 MHz, CDCl₃): δ = 4.02 (d, *J=* 11.8 Hz, 1 H), 3.89 (d, *J=* 11.8 Hz, 1 H), 2.05 (s, 3 H), 1.94 (dtd, *J* = 13.3, 6.3, 2.6 Hz, 1 H), 1.57 - 1.36 (m, 4 H), 1.25 - 1.16 (m, 1 H), 1.12 - 1.06 (m, 1 H), 0.97 (s, 3 H), 0.88 (s, 3 H), 0.80 (d, *J=* 6.7 Hz, 3 H), 0.78 (s, 3 H) ppm.
¹³C-NMR (125 MHz, CDCl₃): δ = 11.8, 16.9, 21.3, 22.2, 23.3, 26.8, 30.9, 32.3, 36.1, 38.3, 41.0, 68.3, 171.3 ppm.
Odor: woody (2), sweet-powdery (2), fresh (2), freesia (2).

### Example 7: Compound (A.3)

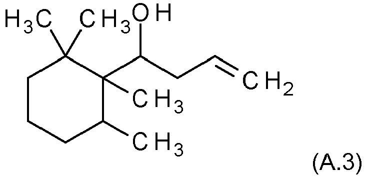

Under N₂-atmosphere, compound (D) (100.0 g, 0.6 mol) was taken up in dry THF (500 mL) and the solution was cooled to 0°C. Allyl magnesium chloride (2 mol/L in hexane, 300 mL) was added slowly while maintaining the temperature in the range of 0 to 5°C. The reaction mixture was stirred at 5°C for 1 h. After the reaction was complete, saturated aqueous NH₄Cl-solution (500 mL) was added and the mixture was stirred at room temperature for 30 minutes. Then, the mixture was filtered and the residue was washed with ethyl acetate (2 x 250 mL). The filtrate was washed with brine (100 mL), phases were separated and the solvent was removed under reduced pressure. 125 g of compound (A.3) were obtained (GC purity 97%, yield 98%).
¹H-NMR (500 MHz, CDCl₃): δ = 5.90 - 5.79 (m, 1 H), 5.14 (br s, 1 H), 5.13 - 5.09 (m, 1 H), 3.75 (d, *J=* 11.1 Hz, 1 H), 2.43 - 2.38 (m, 1 H), 2.27 - 2.14 (m, 2 H), 1.59 - 1.42 (m, 4 H), 1.36 - 1.20 (m, 2 H), 1.08 (s, 3 H), 0.91 (d, *J=* 6.8 Hz, 3 H), 0.86 (s, 3 H), 0.82 (s, 3 H) ppm.
¹³C-NMR (125 MHz, CDCl₃): δ = 137.4, 117.8, 73.3, 44.6, 40.0, 38.7, 37.6, 31.6, 31.0, 27.3, 22.4, 24.3, 18.9, 12.5 ppm.
Odor: woody (3), cedar (3), oak moss (3).

### Example 8: Compound (C.1)

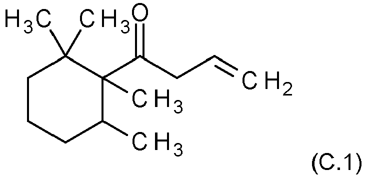

Compound (A.3) (60.0 g, 285 mmol) was dissolved in CH₂Cl₂ (350 mL) and pyridinium chlorochromate (70 g, 325 mmol) was added at 10°C. The reaction mixture was stirred at room temperature for 20 h. After completion of the reaction, the mixture was filtered and the residue was washed with CH₂Cl₂ (2 x 250 mL). The filtrate was extracted with 5 % aqueous NaHCO₃-solution (100 mL) and then with brine (100 mL). Phases were separated and the solvent of the organic phase was removed under reduced pressure. The crude product (60 g) was taken up in heptane (100 mL) and the resulting precipitate was removed by filtration. The solvent was removed under reduced pressure and the desired product was obtained in 85% yield (GC purity 90%).
¹H-NMR (500 MHz, CDCl₃): δ = 5.96 (ddt, *J=* 17.1, 10.2, 6.8 Hz, 1 H), 5.16 - 5.11 (m, 1 H), 5.10 - 5.03 (m, 1 H), 3.24 (d, *J=* 6.8 Hz, 2 H), 2.49 - 2.42 (m, 1 H), 1.55 - 1.48 (m, 4 H), 1.15 (s, 3 H), 1.12 - 1.07 (m, 2 H), 0.94 (s, 3 H), 0.89 (s, 3 H), 0.68 (d, *J* = 6.6 Hz, 3 H) ppm.
¹³C-NMR (125 MHz, CDCl₃): δ = 213.7, 132.1, 117.6, 57.3, 46.5, 36.9, 36.1, 31.7, 29.9, 27.5, 24.4, 21.6, 18.7, 10.7 ppm.
Odor: straw (3), hay, earth-like (3), slightly woody (2).

### Example 9: Compound (A.5)

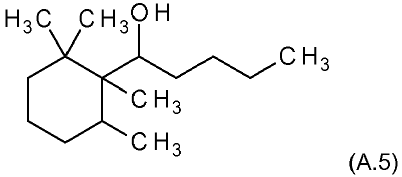

Under N₂ atmosphere, compound (D) (15 g, 90 mmol) was taken up in dry THF (150 mL) and the solution was cooled to -25°C. Butyl lithium (1.6 mol/L in hexanes, 150 mL, 240 mmol) was added over a period of 2 h while maintaining the temperature at -25°C. Then, the reaction mixture was stirred at 5°C for 1 h. The reaction was stopped by addition of 20% aqueous NH₄Cl solution (50 mL) and the mixture was extracted with ethyl acetate (2 x 75 mL). The combined organic phases were washed with brine (50 mL), phases were separated and the solvent was removed under reduced pressure. The product was purified by silica gel chromatography using ethyl acetate : heptane (20:80) as eluent. The desired product was obtained in 38% yield (8 g, GC purity 97%).
¹H-NMR (300 MHz, CDCl₃): δ = 3.71 (d, *J*= 10.6 Hz, 1 H), 2.15 - 2.08 (m, 1 H), 1.60 - 1.21 (m, 12 H), 1.08 (s, 3 H), 0.91 (t, *J* = 7.3 Hz, 3 H), 0.87 (d, *J* = 6.8 Hz, 3 H), 0.85 (s, 3 H), 0.80 (s, 3 H) ppm.
¹³C-NMR (125 MHz, CDCl₃): δ = 75.7, 44.8, 38.8, 37.6, 34.8, 31.6, 31.0, 30.4, 27.3, 24.4, 22.8, 22.5, 18.8, 14.2, 12.7 ppm.

### Example 10: Compound (A.2)

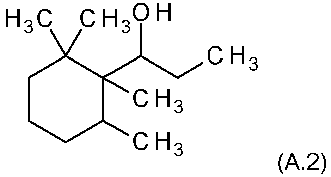

Under N₂ atmosphere, compound (D) (6.8 g, 40 mmol) was taken up in dry THF (50 mL) and the solution was cooled to -25°C. Ethyl lithium (0.5 mol/L in hexane, 100 mL, 50 mmol) was added over a period of 1 h at -25°C. Then, the reaction mixture was stirred at 5°C for 2 h. The reaction was stopped by addition of 20% aqueous NH₄Cl solution (50 mL) and the mixture was extracted with ethyl acetate (2 x 75 mL). The combined organic phases were washed with brine (50 mL), phases were separated and the solvent was removed under reduced pressure. The crude product was purified by silica gel chromatography using ethyl acetate : heptane (20:80) as eluent. The desired product was obtained in 42% yield (3.5 g, GC purity 97%).
¹H-NMR (300 MHz, CDCl₃, one isomer): δ = 3.61 (dd, *J=* 11.1, 2.0 Hz, 1 H), 2.16 - 2.09 (m, 1 H), 1.69 - 1.23 (m, 8 H), 1.08 (s, 3 H), 0.98 (t, *J* = 7.3 Hz, 3 H), 0.87 (d, *J=* 6.8 Hz, 3 H), 0.85 (s, 3 H), 0.80 (s, 3 H) ppm.
¹³C-NMR (125 MHz, CDCI3, one isomer): δ = 77.3, 44.9, 38.7, 37.5, 31.6, 31.0, 27.6, 27.3, 24.4, 22.5, 18.8, 12.5, 12.4 ppm.
Odor: ambra (1), naphthalene.

### Example 11: Compound (C.3)

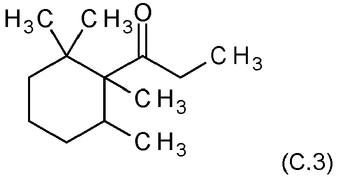

Obtained by oxidation of compound (A.2) in analogy to example 8.
¹H-NMR (300 MHz, CDCl₃, one isomer): δ = 2.5 (m, 3 H), 1.69 - 1.23 (m, 6 H), 1.08 (s, 3 H), 0.98 (t, *J=* 7.3 Hz, 3 H), 0.87 (d, *J=* 6.8 Hz, 3 H), 0.85 (s, 3 H), 0.80 (t, 3 H) ppm.
¹³C-NMR (125 MHz, CDCl₃): δ = 216.4, 57.0, 39.1, 36.0, 35.0, 31.8, 29.9, 27.5, 24.5, 21.6, 18.7, 10.7, 8.2 ppm.
Odor: earth-like (3), cedar wood (3), powdery, dry-

### Example 12: Compound (C.2)

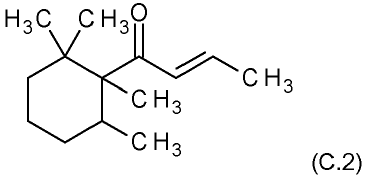

Compound (C.1) (7 g, 33 mmol) was dissolved in methanol (50 mL) at room temperature. Triethylamine (12 g, 117 mmol) was added. Stirring was continued at 25°C for 20 h. Solvent was distilled out under reduced pressure to give the crude compound (7.0 g). Then, the crude compound was diluted with dichloromethane (50 mL) and extracted with aqueous HCI (1 mol/L, 30 mL) followed by water (50 mL). The organic phase was dried over Na₂SO₄ and the solvent was evaporated under reduced pressure (60°C, 100 mbar). The product was purified by column chromatography (eluent heptane : ethyl acetate 98:2) and obtained in 30% yield (2 g, GC purity 95%).
¹H-NMR (500 MHz, CDCl₃): δ = 6.9-6.7 (m, 1 H), 5.5 - 5.4 (d, 1 H), 2.49 - 2.42 (m, 1 H), 1.9-1.8 (d, 3 H), 1.55 - 1.48 (m, 4 H), 1.15 (s, 3 H), 1.12 - 1.07 (m, 2 H), 0.94 (s, 3 H), 0.89 (s, 3 H), 0.68 (d, *J=* 6.6 Hz, 3 H) ppm.
¹³C-NMR (125 MHz, CDCl₃): δ = 203.9, 140.7, 129.4, 55.7, 37.0, 36.1, 31.5, 30.0, 27.3, 24.3, 21.8, 18.4, 18.3, 10.7 ppm.
Odor: dried fruit (3), damascone (3), rosy (3), lovage (2).

### Example 13: Compound (C.4)

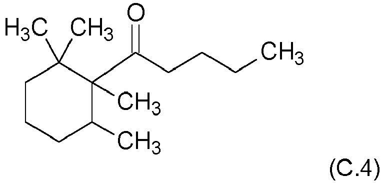

Obtained by oxidation of compound (A.5) in analogy to example 8.
¹H-NMR (500 MHz, CDCl₃): δ = 2.47 - 2.36 (m, 3 H), 1.55 - 1.42 (m, 6 H), 1.33 - 1.21 (m, 2 H), 1.12 (s, 3 H), 1.11 - 1.03 (m, 2 H), 0.90 (s, 3 H), 0.89 (t, J= 7.3 Hz, 3 H), 0.86 (s, 3 H), 0.63 (d, *J=* 6.6 Hz, 3 H) ppm.
¹³C-NMR (125 MHz, CDCl₃): δ = 215.9, 56.9, 41.8, 37.0, 36.0, 31.8, 29.9, 27.6, 26.2, 24.5, 22.5, 21.6, 18.7, 14.1, 10.7 ppm.

### Example 14: Compound (B.2)

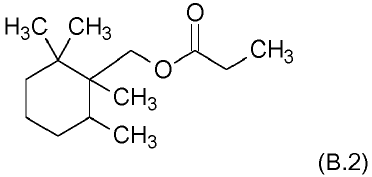

Propionyl chloride (1.3 g, 13 mmol) was added slowly at 5°C to a solution of compound (A.1) (2.0 g, 12 mmol) and triethylamine (1.75 g, 17 mmol) in dichloromethane (10 mL). The reaction mixture was stirred at RT for 3 h. Then, water (10 mL) was added and the phases were separated. The organic phase was washed with 1N HCl (10 mL) followed by water (10 mL) and the solvent was removed under reduced pressure. The product was purified by column chromatography (2.0 g, 75%).
¹H-NMR (500 MHz, CDCl₃): δ = 4.00 (d, *J=* 11.8 Hz, 1 H), 3.89 (d, *J=* 11.8 Hz, 1 H), 2.31 (q, *J* = 7.8 Hz, 2 H), 1.98 - 1.90 (m, 1 H), 1.56 - 1.35 (m, 4 H), 1.24 - 1.16 (m, 1 H), 1.13 (t, *J* = 7.6 Hz, 3 H), 1.09 - 1.07 (m, 1 H), 0.96 (s, 3 H), 0.87 (s, 3 H), 0.79 (d, *J* = 6.7 Hz, 3 H), 0.76 (s, 3 H) ppm.
¹³C-NMR (125 MHz, CDCl₃): δ = 174.5, 68.1, 41.1, 38.3, 36.1, 32.3, 31.0, 28.0, 26.8, 23.4, 22.3, 16.9, 11.9, 9.2 ppm.

## Claims

1. A compound of formula (A) 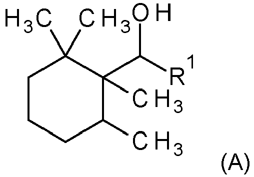
wherein R¹ is selected from C₁-C₈-alkyl, and C₂-C₈-alkenyl,
and esters of the compound of formula (A),
and ketones of the compound of formula (A),
with the proviso that R¹ can also be hydrogen, if compound (A) is an ester of formula (A); and
with the proviso that compound 1-(1,2,2,6-tetramethylcyclohexyl)ethanone is excluded.

2. The compounds of claim 1, wherein the esters of the compound of formula (A) are selected from compounds of formula (B), 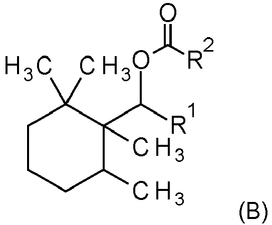 wherein
R¹ is selected from hydrogen, C₁-C₈-alkyl, and C₂-C₈-alkenyl,
R² is selected from C₁-C₅-alkyl.

3. The compounds of claim 1, wherein the ketones of the compound of formula (A) are selected from compounds of formula (C), 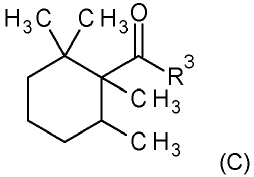 wherein R³ is selected from C₁-C₈-alkyl, and C₂-C₈-alkenyl, with the proviso that R³ is methyl is excluded.

4. The compound according to claim 1, which is selected from

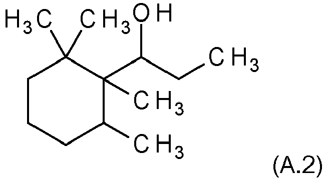

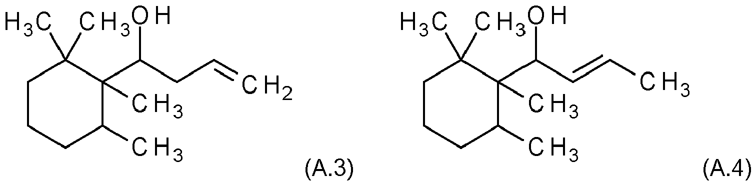

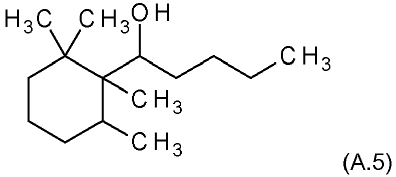

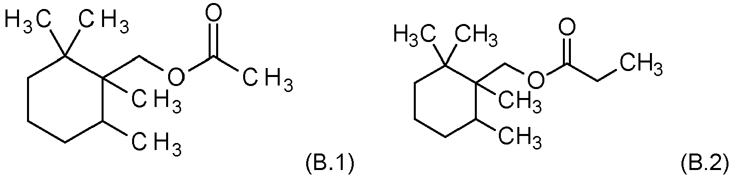

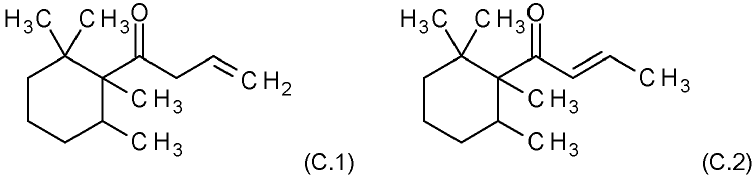

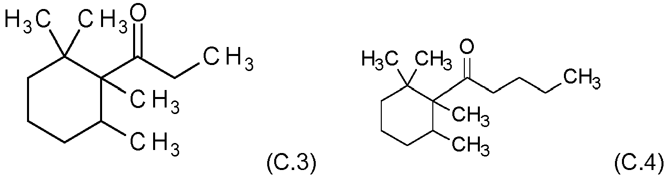

5. A method for preparing compounds of formula (A) 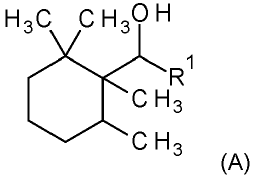 wherein R¹ is selected from hydrogen, C₁-C₈-alkyl, and C₂-C₈-alkenyl, comprising
i) providing a compound of formula (D), 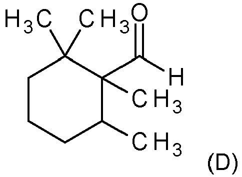 and either
iia) reacting the compound of formula (D) with a reducing agent, which is selected from hydride compounds, or by catalytic hydrogenation, to obtain a compound of formula (A.a), 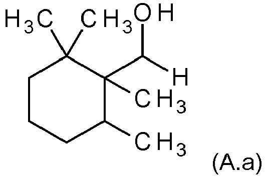 or
iib) reacting the compound of formula (D) with a nucleophilic agent, which is selected from metal C₁-C₈-alkyl compounds and metal C₂-C₈-alkenyl compounds, to obtain a compound of formula (A.b)

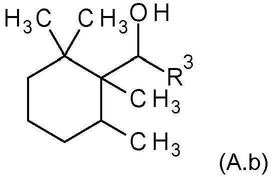

6. The method according to claim 5, further comprising
iiia) reacting the compound of formula (A.a) with a compound selected from ketenes, saturated and unsaturated monocarboxylic acids, halides of saturated and unsaturated monocarboxylic acids, anhydrides of saturated and unsaturated monocarboxylic acids, and C₁-C₄-alkyl esters of saturated and unsaturated monocarboxylic acids to obtain a compound of formula (B.a). 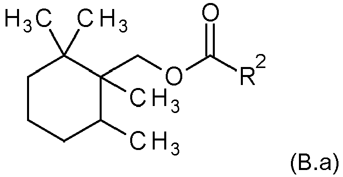 wherein R² is selected from C₁-C₅-alkyl,

7. The method according to claim 5, for preparing a compound of formula (A.b), wherein in step iib), the nucleophilic agent is selected from metal C₁-C₈-alkyl compounds and metal C₂-C₈-alkenyl compounds, which comprise at least one ligand R³.

8. The method according to any one of claims 5 or 7, further comprising
iiib) reacting the compound of formula (A.b) with an oxidation reagent to obtain a compound of formula (C) 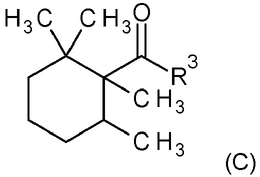 wherein R³ is selected from C₁-C₈-alkyl, and C₂-C₈-alkenyl.

9. The method according to any one of the claims 5 to 8, wherein step i) comprises the following steps:
ia) providing a compound of formula (D.a), 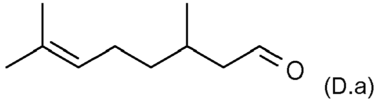
ib) subjecting the compound of formula (D.a) to an aldol condensation reaction with formaldehyde to obtain a compound of formula (D.b), 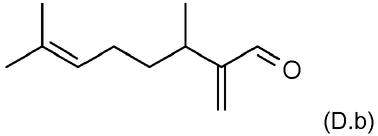
ic) subjecting the compound of formula (D.b) to a hydrogenation reaction to obtain a compound of formula (D.c), 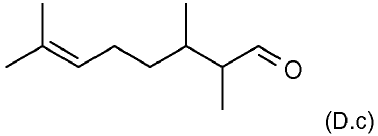
id) subjecting the compound of formula (D.c) to an acetylation reaction to obtain a compound of formula (D.d), 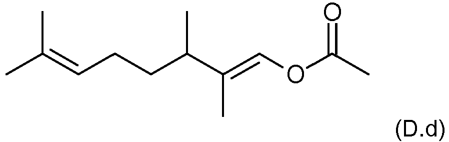
ie) subjecting the compound of formula (D.d) to a cyclisation reaction to obtain the compound of formula (D).

10. The use of a compound of formula (A) 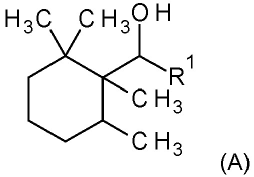
wherein R¹ is selected from C₁-C₈-alkyl, and C₂-C₈-alkenyl,
or of an ester of the compound of formula (A),
or of a ketone of the compound of formula (A),
with the proviso that R¹ can also be hydrogen, if compound (A) is an ester of formula (A),
or of the compound of formula A.1 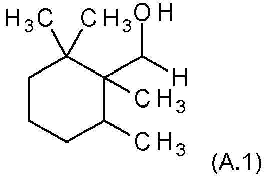
or of the compound of formula D 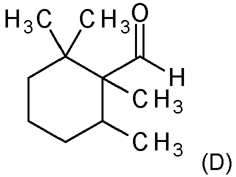 as aroma chemical; where the compound is in particular defined as in any one of claims 1 to 3.

11. A flavoring composition comprising
a) at least one compound as defined in any one of claims 1 to 3 or compound (D) 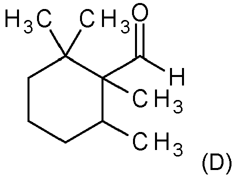 and
b) optionally at least one further aroma chemical different from the compounds of component a), and
c) optionally at least one diluent,
with the proviso that the composition comprises at least one of the components b) or c).

12. A perfumed or aromatized product,
- comprising an organoleptically effective amount of at least one compound of the formula (A) as defined in claim 10 or an ester thereof or a ketone thereof or compound (A.1) as defined in claim 10 or compound (D) as defined in claim 10 or 11; in particular comprising an organoleptically effective amount of at least one compound as defined as in any one of claims 1 to 3; and/or
- comprising an organoleptically effective amount of at least one flavoring composition as defined in claim 11.

13. A perfumed or aromatized product according to claim 12, wherein the product is selected from perfumes, detergents and cleaners, cosmetic compositions, bodycare compositions, hygiene articles, products for oral and dental hygiene, scent dispensers, fragrances, pharmaceutical compositions and crop protection compositions.

14. A method for imparting and/or intensifying an odor or taste of a product which the product is brought into contact with an organoleptically effective amount of at least one compound of the formula (A) as defined in claim 10, of an ester thereof or of a ketone thereof, or compound (D) as defined in claim 10 or 11 or of compound (A.1) as defined in claim 10; where in particular the product is brought into contact with an organoleptically effective amount of at least one compound as defined in any of claims 1 to 3.

15. The use of a compound selected from
- the compound of formula (A.1) 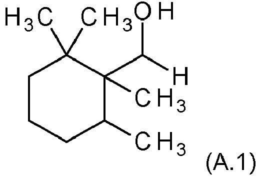 with an aroma of eucalyptol, flowery, earth-like,
- the compound of formula (B.1) 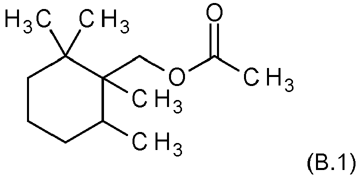 with an aroma of woody, sweet-powdery, fresh, freesia,
- the compound of formula (A.3) 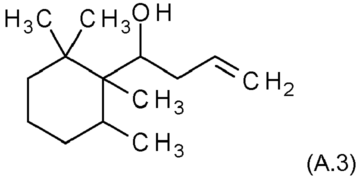 with an aroma of woody, cedar, oak moss,
- the compound of formula (C.1) 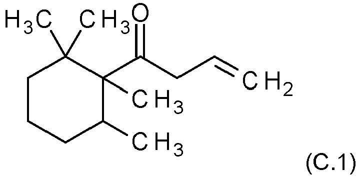 with an aroma of straw, hay, earth-like, slightly woody,
- the compound of formula (A.2) 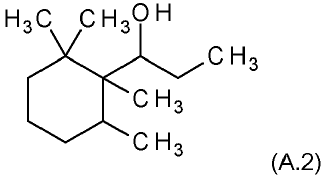 with an aroma of ambra, naphthalene,
- the compound of formula (C.3) 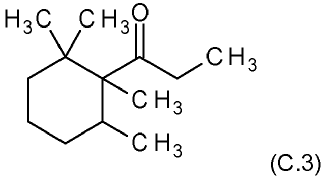 with an aroma of earth-like, cedar wood, powdery, dry,
- the compound of formula (C.2) 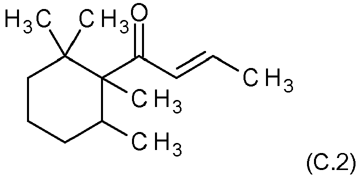 with an aroma of dried fruit, damascone, rosy, lovage,
- the compound of formula (D) as defined in claim 10 or 11 with an aroma of eucalyptol, campher, citrus, spruce needle,
as aroma chemical.

## Patentansprüche

1. Verbindung der Formel (A) 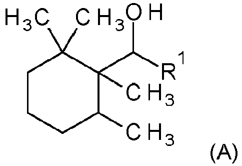
worin R¹ aus C₁-C₈-Alkyl und C₂-C₈-Alkenyl ausgewählt ist,
und Ester der Verbindung der Formel (A) und Ketone der Verbindung der Formel (A),
mit der Maßgabe, dass R¹ auch für Wasserstoff stehen kann, wenn es sich bei Verbindung (A) um einen Ester der Formel (A) handelt; und
mit der Maßgabe, dass die Verbindung 1-(1,2,2,6-Tetramethylcyclohexyl)ethanon ausgeschlossen ist.

2. Verbindungen nach Anspruch 1, wobei die Ester der Verbindung der Formel (A) aus Verbindungen der Formel (B) ausgewählt sind, 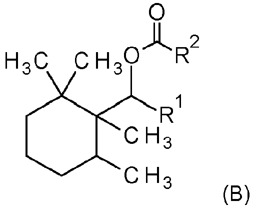 worin
R¹ aus Wasserstoff, C₁-C₈-Alkyl und C₂-C₈-Alkenyl ausgewählt ist,
R² aus C₁-C₅-Alkyl ausgewählt ist.

3. Verbindungen nach Anspruch 1, wobei die Ketone der Verbindung der Formel (A) aus Verbindungen der Formel (C) ausgewählt sind, 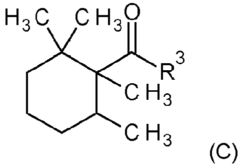 worin R³ aus C₁-C₈-Alkyl und C₂-C₈-Alkenyl ausgewählt ist, mit der Maßgabe, dass R³ gleich Methyl ausgeschlossen ist.

4. Verbindung nach Anspruch 1, die aus 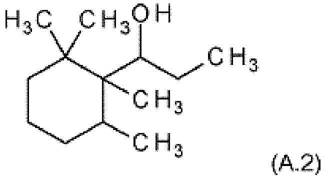 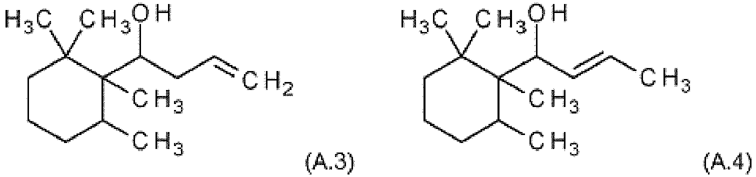 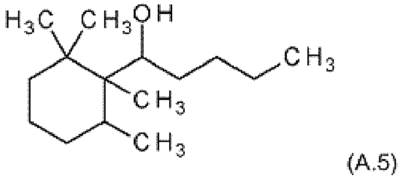 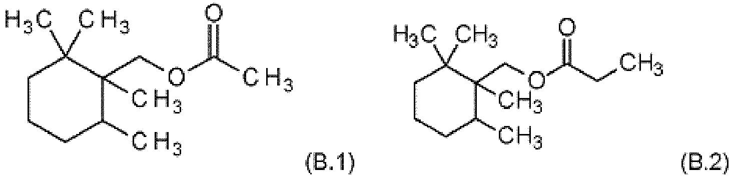 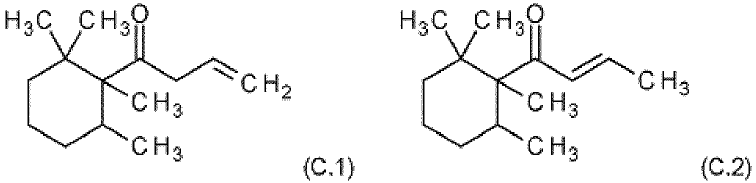 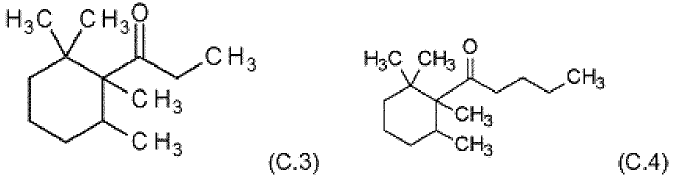 ausgewählt ist.

5. Verfahren zur Herstellung von Verbindungen der Formel (A) 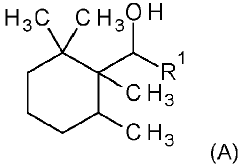 worin R¹ aus Wasserstoff, C₁-C₈-Alkyl und C₂-C₈-Alkenyl ausgewählt ist,
bei dem man
i) eine Verbindung der Formel (D), 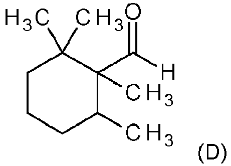 bereitstellt und entweder
iia) die Verbindung der Formel (D) mit einem aus Hydridverbindungen ausgewählten Reduktionsmittel oder durch katalytische Hydrierung zu einer Verbindung der Formel (A.a), 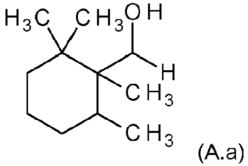 umsetzt
oder
iib) die Verbindung der Formel (D) mit einem aus Metall-C₁-C₈-alkylverbindungen und Metall-C₂-C₈-alkenylverbindungen ausgewählten nucleophilen Agens zu einer Verbindung der Formel (A.b) 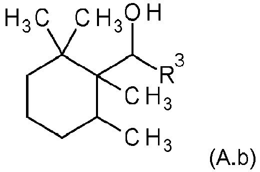 umsetzt.

6. Verfahren nach Anspruch 5, bei dem man ferner
iiia) die Verbindung der Formel (A.a) mit einer aus Ketenen, gesättigten und ungesättigten Monocarbonsäuren, Halogeniden von gesättigten und ungesättigten Monocarbonsäuren, Anhydriden von gesättigten und ungesättigten Monocarbonsäuren und C₁-C₄-Alkylestern von gesättigten und ungesättigten Monocarbonsäuren zu einer Verbindung der Formel (B.a), 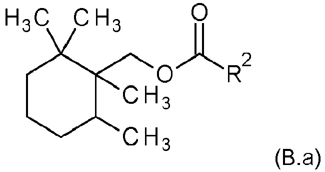 worin R² aus C₁-C₅-Alkyl ausgewählt ist, umsetzt.

7. Verfahren nach Anspruch 5 zur Herstellung einer Verbindung der Formel (A.b), bei dem man in Schritt iib) das nucleophile Agens aus Metall-C₁-C₈-alkylverbindungen und Metall-C₂-C₈-alkenyl-verbindungen mit mindestens einem Liganden R³ auswählt.

8. Verfahren nach einem der Ansprüche 5-7, bei dem man ferner
iiib) die Verbindung der Formel (A.b) mit einem Oxidationsreagenz zu einer Verbindung der Formel (C) 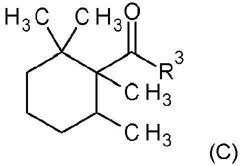 worin R³ aus C₁-C₈-Alkyl und C₂-C₈-Alkenyl ausgewählt ist,
umsetzt.

9. Verfahren nach einem der Ansprüche 5 bis 8, bei dem man in Schritt i):
ia) die Verbindung der Formel (D.a), 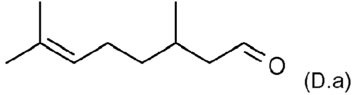 bereitstellt,
ib) die Verbindung der Formel (D.a) einer Aldolkondensationsreaktion mit Formaldehyd zu einer Verbindung der Formel (D.b), 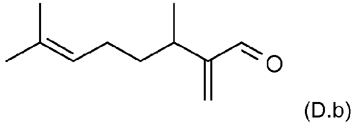 unterwirft,
ic) die Verbindung der Formel (D.b) eine Hydrierungsreaktion zu einer Verbindung der Formel (D.c), 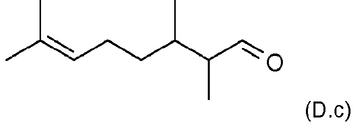 unterwirft,
id) die Verbindung der Formel (D.c) eine Acetylierungsreaktion zu einer Verbindung der Formel (D.d), 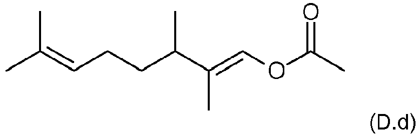 unterwirft,
ie) die Verbindung der Formel (D.d) eine Cyclisierungsreaktion zu der Verbindung der Formel (D) unterwirft.

10. Verwendung einer Verbindung der Formel (A) 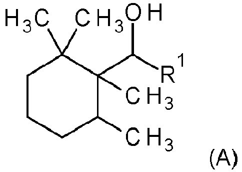
worin R¹ aus C₁-C₈-Alkyl und C₂-C₈-Alkenyl ausgewählt ist,
oder eines Esters der Verbindung der Formel (A)
oder eines Ketons der Verbindung der Formel (A),
mit der Maßgabe, dass R¹ auch für Wasserstoff stehen kann, wenn es sich bei Verbindung (A) um einen Ester der Formel (A) handelt, oder der Verbindung der Formel A.1 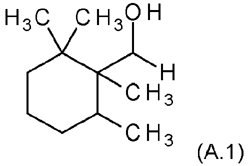
oder der Verbindung der Formel D 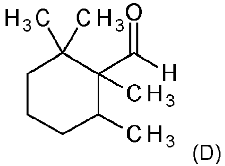 als Aromachemikalie; wobei die Verbindung insbesondere wie in einem der Ansprüche 1 bis 3 definiert ist.

11. Geschmacksstoffzusammensetzung, umfassend
a) mindestens eine Verbindung gemäß einem der Ansprüche 1 bis 3 oder Verbindung (D) 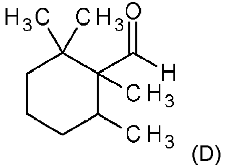 und
b) gegebenenfalls mindestens eine weitere Aromachemikalie, die von den Verbindungen der Komponente a) verschieden sind, und
c) gegebenenfalls mindestens ein Verdünnungsmittel,
mit der Maßgabe, dass die Zusammensetzung mindestens eine der Komponenten b) oder c) umfasst.

12. Parfümiertes oder aromatisiertes Produkt,
- umfassend eine organoleptisch wirksame Menge mindestens einer Verbindung der Formel (A) gemäß Anspruch 10 oder eines Esters davon oder eines Ketons davon oder Verbindung (A.1) gemäß Anspruch 10 oder von Verbindung (D) gemäß Anspruch 10 oder 11; insbesondere umfassend eine organoleptisch wirksame Menge mindestens eine Verbindung gemäß einem der Ansprüche 1 bis 3; und/oder
- umfassend eine organoleptisch wirksame Menge mindestens einer Geschmacksstoffzusammensetzung gemäß Anspruch 11.

13. Parfümiertes oder aromatisiertes Produkt nach Anspruch 12, wobei das Produkt aus Parfüms, Wasch- und Reinigungsmitteln, kosmetischen Mitteln, Körperpflegemitteln, Hygieneartikeln, Produkten für die Mund- und Zahnhygiene, Duftspender, Duftstoffen, pharmazeutischen Mitteln und Pflanzenschutzmitteln ausgewählt ist.

14. Verfahren zum Verleihen und/oder Verstärken eines Geruchs oder Geschmacks eines Produkts, bei dem man das Produkt mit einer organoleptisch wirksamen Menge mindestens einer Verbindung der Formel (A) gemäß Anspruch 10, eines Esters davon oder eines Ketons davon oder von Verbindung (D) gemäß Anspruch 10 oder 11 oder von Verbindung (A.1) gemäß Anspruch 10 in Kontakt bringt; wobei man insbesondere das Produkt mit einer organoleptisch wirksamen Menge mindestens einer Verbindung gemäß einem der Ansprüche 1 bis 3 in Kontakt bringt.

15. Verwendung einer Verbindung, ausgewählt aus
- der Verbindung der Formel (A.1) 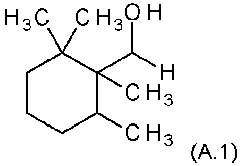 mit dem Aroma Eucalyptol, blumig, erdig,
- der Verbindung der Formel (B. 1) 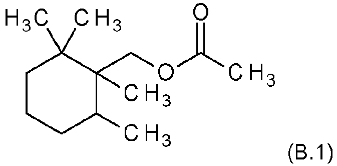 mit dem Aroma holzig, süß-pulverig, frisch, Freesie,
- der Verbindung der Formel (A.3) 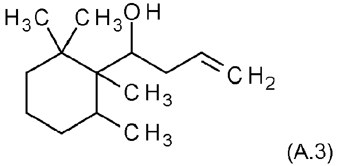 mit dem Aroma holzig, Zeder, Eichenmoos,
- der Verbindung der Formel (C.1) 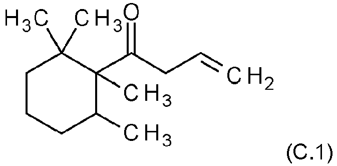 mit dem Aroma Stroh, Heu, erdig, leicht holzig,
- der Verbindung der Formel (A.2) 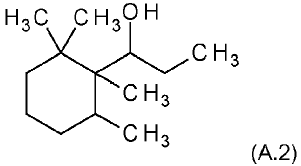 mit dem Aroma Ambra, Naphthalin,
- der Verbindung der Formel (C.3) 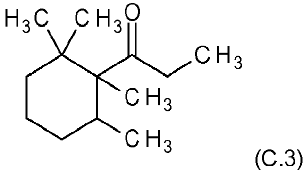 mit dem Aroma erdig, Zedernholz, pulverig, trocken,
- der Verbindung der Formel (C.2) 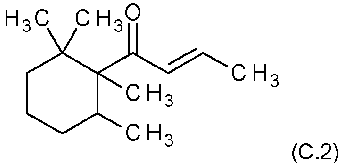 mit dem Aroma getrocknete Früchte, Damascon, rosig, Liebstöckel,
- der Verbindung der Formel (D) gemäß Anspruch 10 oder 11 mit dem Aroma Eucalyptol, Campher, Zitrus, Fichtennadel
als Aromachemikalie.

## Revendications

1. Composé de formule (A) 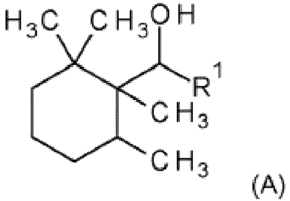
R¹ étant choisi parmi C₁₋₈-alkyle, et C₂₋₈-alcényle,
et esters du composé de formule (A),
et cétones du composé de formule (A),
étant entendu que R¹ peut également être hydrogène, si le composé (A) est un ester de formule (A) ; et étant entendu que le composé 1-(1,2,2,6-tétraméthylcyclohexyl)éthanone est exclu.

2. Composés selon la revendication 1, les esters du composé de formule (A) étant choisis parmi des composés de formule (B), 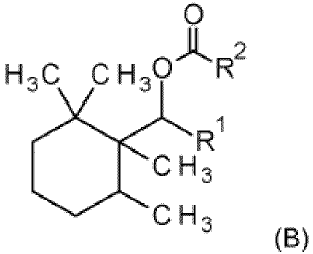
R¹ étant choisi parmi hydrogène, C₁₋₈-alkyle, et C₂₋₈-alcényle,
R² étant choisi parmi C₁₋₅-alkyle.

3. Composés selon la revendication 1, les cétones du composé de formule (A) étant choisies parmi des composés de formule (C), 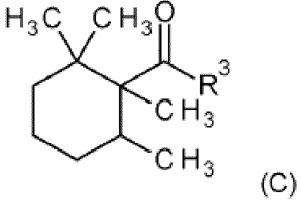 R³ étant choisi parmi C₁₋₈-alkyle, et C₂₋₈-alcényle, étant entendu que R³ étant méthyle est exclu.

4. Composé selon la revendication 1, qui est choisi parmi

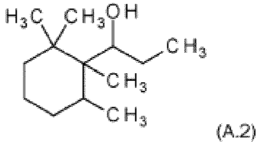

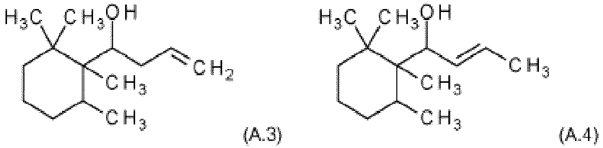

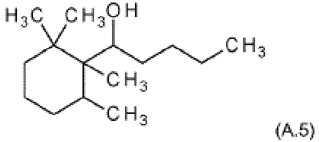

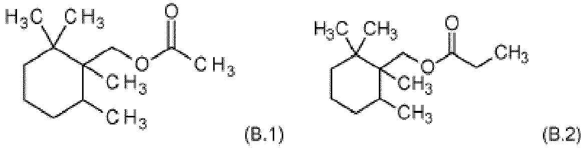

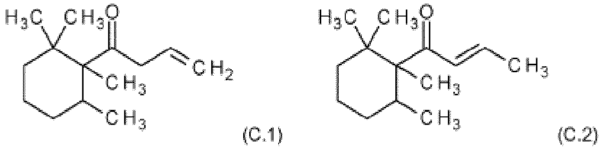

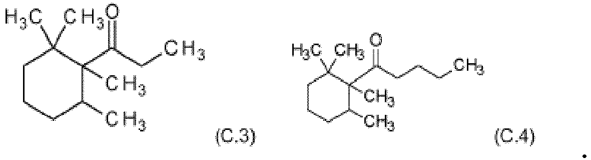

5. Procédé pour la préparation de composés de formule (A) 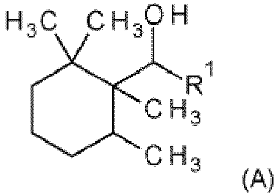 R¹ étant choisi parmi hydrogène, C₁₋₈-alkyle, et C₂₋₈-alcényle,
comprenant
i) la mise à disposition d'un composé de formule (D) 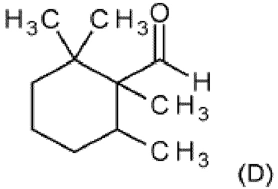 et soit
iia) la mise en réaction du composé de formule (D) avec un agent de réduction, qui est choisi parmi des composés de type hydrure, ou par hydrogénation catalytique, pour obtenir un composé de formule (A.a), 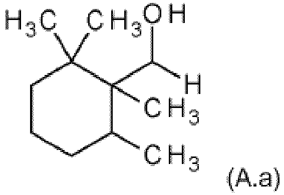 soit
iib) la mise en réaction du composé de formule (D) avec un agent nucléophile, qui est choisi parmi des composés de type C₁₋₈-alkyl-métal et des composés de type C₂₋₈-alcényl-métal, pour obtenir un composé de formule (A.b)

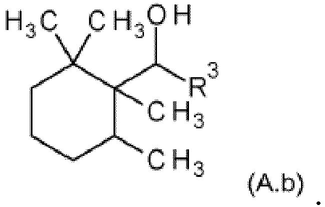

6. Procédé selon la revendication 5, comprenant en outre
iiia) la mise en réaction du composé de formule (A.a) avec un composé choisi parmi des cétènes, des acides monocarboxyliques saturés et insaturés, des halogénures d'acides monocarboxyliques saturés et insaturés, des anhydrides d'acides monocarboxyliques saturés et insaturés, et des esters de C₁₋₄-alkyle d'acides monocarboxyliques saturés et insaturés pour obtenir un composé de formule (B.a), 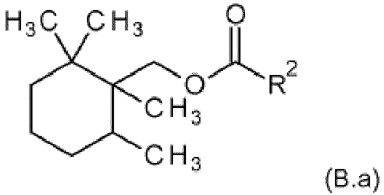 R2 étant choisi parmi C₁₋₅-alkyle.

7. Procédé selon la revendication 5, pour la préparation d'un composé de formule (A.b), dans lequel dans l'étape iib), l'agent nucléophile est choisi parmi des composés de type C₁₋₈-alkyl-métal et des composés de type C₂₋₈-alcényl-métal, qui comprennent au moins un ligand R³.

8. Procédé selon l'une quelconque des revendications 5 et 7, comprenant en outre
iiib) la mise en réaction du composé de formule (A.b) avec un réactif d'oxydation pour obtenir un composé de formule (C) 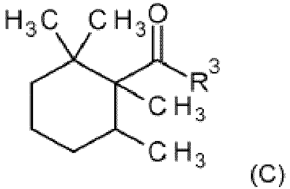 R³ étant choisi parmi C₁₋₈-alkyle, et C₂₋₈-alcényle.

9. Procédé selon l'une quelconque des revendications 5 à 8, l'étape i) comprenant les étapes suivantes :
ia) mise à disposition d'un composé de formule (D.a), 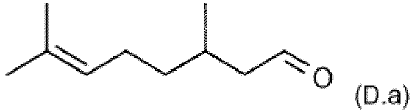
ib) soumission du composé de formule (D.a) à une réaction de condensation aldol avec du formaldéhyde pour obtenir un composé de formule (D.b), 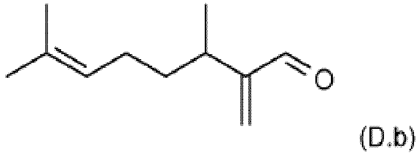
ic) soumission du composé de formule (D.b) à une réaction d'hydrogénation pour obtenir un composé de formule (D.c), 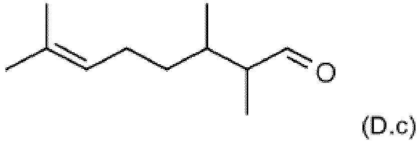
id) soumission du composé de formule (D.c) à une réaction d'acétylation pour obtenir un composé de formule (D.d), 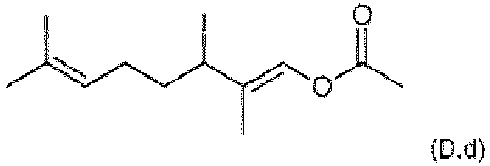
ie) soumission du composé de formule (D.d) à une réaction de cyclisation pour obtenir le composé de formule (D).

10. Utilisation d'un composé de formule (A) 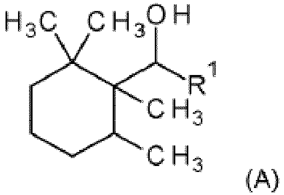
R¹ étant choisi parmi C₁₋₈-alkyle, et C₂₋₈-alcényle,
ou d'un ester du composé de formule (A),
ou d'une cétone du composé de formule (A),
étant entendu que R¹ peut également être hydrogène, si le composé (A) est un ester de formule (A),
ou du composé de formule A.1 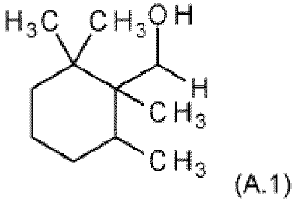
ou du composé de formule D 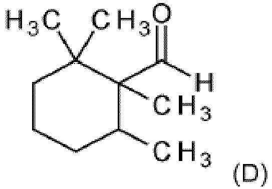 en tant qu'arôme chimique ; dans laquelle le composé est en particulier défini comme dans l'une quelconque des revendications 1 à 3.

11. Composition d'arôme comprenant
a) au moins un composé tel que défini dans l'une quelconque des revendications 1 à 3 ou le composé (D) 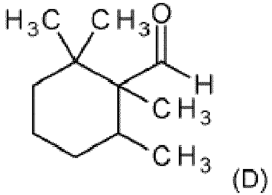 et
b) éventuellement au moins un arôme chimique supplémentaire différent des composés du composant a), et
c) éventuellement au moins un diluant,
étant entendu que la composition comprend au moins l'un des composants b) et c).

12. Produit parfumé ou aromatisé,
- comprenant une quantité efficace sur le plan organoleptique d'au moins un composé de formule (A) tel que défini dans la revendication 10 ou d'un ester correspondant ou d'une cétone correspondante ou du composé (A.1) tel que défini dans la revendication 10 ou du composé (D) tel que défini dans la revendication 10 ou 11 ; en particulier comprenant une quantité efficace sur le plan organoleptique d'au moins un composé tel que défini dans l'une quelconque des revendications 1 à 3 ; et/ou
- comprenant une quantité efficace sur le plan organoleptique d'au moins une composition telle que définie dans la revendication 11.

13. Produit parfumé ou aromatisé selon la revendication 12, le produit étant choisi parmi des parfums, des détergents et des nettoyants, des compositions cosmétiques, des compositions de soin corporel, des articles hygiéniques, des produits pour l'hygiène buccale et dentaire, des diffuseurs de parfum, des fragrances, des compositions pharmaceutiques et des compositions de protection de cultures.

14. Procédé pour conférer et/ou intensifier une odeur ou un goût d'un produit, dans lequel le produit est mis en contact avec une quantité efficace sur le plan organoleptique d'un composé de formule (A) tel que défini dans la revendication 10, d'un ester correspondant ou d'une cétone correspondante, ou du composé (D) tel que défini dans la revendication 10 ou 11 ou du composé (A.1) tel que défini dans la revendication 10 ; dans lequel en particulier le produit est mis en contact avec une quantité efficace sur le plan organoleptique d'au moins un composé tel que défini dans l'une quelconque des revendications 1 à 3.

15. Utilisation d'un composé choisi parmi
- le composé de formule (A.1) 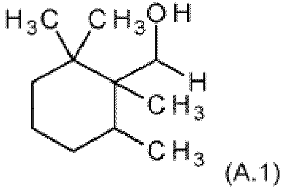 doté d'un arôme d'eucalyptol, fleuri, similaire à la terre,
- le composé de formule (B.1) 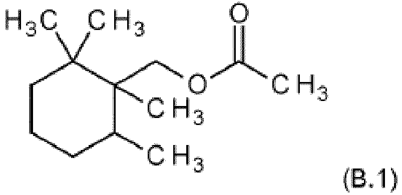 doté d'un arôme boisé, sucré-poudreux, frais, de freesia,
- le composé de formule (A.3) 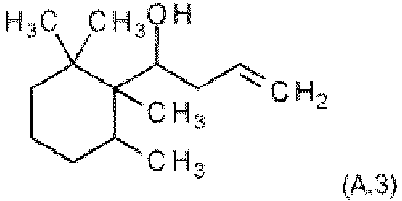 doté d'un arôme boisé, de cèdre, de mousse de chêne,
- le composé de formule (C.1) 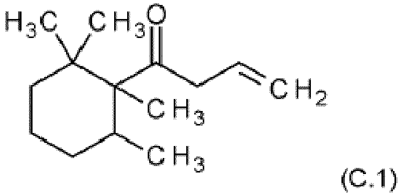 doté d'un arôme de paille, de foin, similaire à la terre, légèrement boisé,
- le composé de formule (A.2) 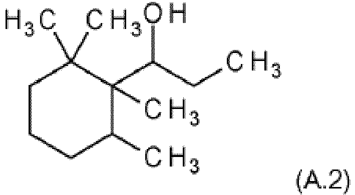 doté d'un arôme d'ambre, de naphtalène,
- le composé de formule (C.3) 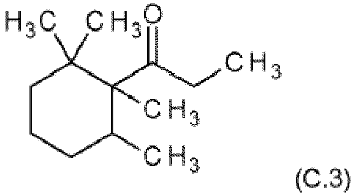 doté d'un arôme similaire à la terre, de bois de cèdre, poudreux, sec,
- le composé de formule (C.2) 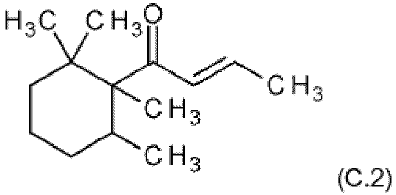 doté d'un arôme de fruit sec, de damascone, rosé, de livèche,
- le composé de formule (D) tel que défini dans la revendication 10 ou 11 doté d'un arôme d'eucalyptol, de camphre, d'agrume, d'aiguille d'épinette,
en tant qu'arôme chimique.
